# EUROPÄISCHE PATENTSCHRIFT

(11) **EP 3 595 508 B1**
(45) Veröffentlichungstag und Bekanntmachung des Hinweises auf die Patenterteilung: **26.10.2022**
(21) Anmeldenummer: 18707308.5
(22) Anmeldetag: 16.02.2018
(51) Int. Cl.: A61B 5/00, A61B 5/053

(54) **VERFAHREN UND VORRICHTUNG ZUM ERMITTELN EINES HAUTFEUCHTIGKEITSGEHALTS**
METHOD AND DEVICE FOR DETERMINING SKIN MOISTURE CONTENT
PROCÉDÉ ET DISPOSITIF DE DÉTERMINATION DE LA TENEUR EN HUMIDITÉ CUTANÉE

(30) Priorität: 17.03.2017 DE 102017204491
(43) Veröffentlichungstag der Anmeldung: 22.01.2020
(73) Patentinhaber: Henkel AG & Co. KGaA, 40589 Düsseldorf (DE)
(72) Erfinder: BOCK, Andreas, 41464 Neuss (DE); WELSS, Thomas, 40591 Düsseldorf (DE); HUNDEIKER, Claudia, 40667 Meerbusch (DE); WALDMANN-LAUE, Marianne, 40789 Monheim (DE)
(86) Internationale Anmeldenummer: PCT/EP2018/053864
(87) Internationale Veröffentlichungsnummer: WO 2018/166749

(56) Entgegenhaltungen:
- WO-A1-2013/033724
- WO-A1-2013/116316
- WO-A1-2016/012469
- WO-A1-2016/069788
- WO-A2-2010/107913
- US-A1- 2011 301 441
- US-A1- 2014 330 136
- US-A1- 2017 086 741

## Beschreibung

Verschiedene Ausführungsformen betreffen im Allgemeinen ein Verfahren und eine Vorrichtung zum Ermitteln eines Hautfeuchtigkeitsgehalts und ein Verfahren zum Ermitteln einer kosmetischen Hautbehandlungsempfehlung.

Eine ausgewogene Versorgung von Haut mit Feuchtigkeit kann entscheidend sein für eine jugendlich strahlende Erscheinung der Haut. Ein Gehalt an in der Haut gebundenem Wasser (auch als Hautfeuchtigkeitsgehalt bezeichnet) kann mit dem Alter kontinuierlich abnehmen. Die Haut kann dann trocken und schlaff erscheinen und ihre Ausstrahlungskraft verlieren.

Der Hautfeuchtigkeitsgehalt kann durch eine Vielzahl an unterschiedlichen Technologien mittels Pflegeprodukten verbessert werden. Physiologisch relevant können insbesondere Technologien sein, bei welchen Wirksubstanzen in die Haut eindringen (penetrieren) und in der Epidermis und der Dermis eine Bindung von Wasser optimieren.

Eine Möglichkeit einer zeitnahen Erfassung von Mangelzuständen in der Hautfeuchte kann es dem Nutzer ermöglichen, sofort auf eine Mangelsituation zu reagieren.

Ohne fachkundige dermatologische oder kosmetische Beratung kann es für einen Nutzer schwer sein, seinen individuellen Hautzustand (z.B. Hautfeuchtigkeitsgehalt) und die für seinen Hautzustand (z.B. Hautfeuchtigkeitsgehalt) geeigneten Kosmetika zu ermitteln.

Sofern der Nutzer dennoch kosmetische Produkte konsumiert, kann es praktisch unmöglich sein, einen Behandlungserfolg nachzuvollziehen, weil es dem Nutzer, z.B. zu Hause, an Möglichkeiten fehlt, standardisiert und objektiv ein Behandlungsergebnis zu beurteilen.

Dadurch kann es dem Konsumenten erschwert sein, eine individuelle Wirksamkeit eines Kosmetikums zu beurteilen, was dazu führen kann, dass eine Motivation, eine entsprechende kosmetische Behandlung, beispielsweise längerfristig, durchzuführen, beeinträchtigt sein kann. Dies kann selbst dann der Fall sein, wenn ein Kosmetikprodukt geeignet wäre, eine objektiv nachweisbare erwünschte Wirkung zu erzielen.

Dokument WO2016012469 A1 beschreibt ein optisches Verfahren zur Bestimmung der Hautfeuchtigkeit und des Sauerstoffgehalts im Blut auf Basis der Photoplethysmographie.

In vielen Bereichen des täglichen Lebens gibt es seit einiger Zeit einen Trend zu personalisierten Programmen, die auf individuelle Voraussetzungen und Bedürfnisse gezielt eingehen können, beispielsweise in einem Ernährungs- oder Gesundheitsbereich, aber auch in einem Bereich personalisierter Kosmetik. Diese kann es einem Nutzer ermöglichen, gezielt Kosmetikprodukte zu finden und/oder Pflegehinweise zu erhalten, die auf individuelle Bedürfnisse seiner Haut abgestimmt sind, und somit eine besonders hohe Wirksamkeit ermöglichen.

Es werden eine Vorrichtung und ein Verfahren zum Ermitteln eines Hautfeuchtigkeitsgehalts bereitgestellt.

Das Ermitteln des Hautfeuchtigkeitsgehalts in der Haut und ein kontinuierliches Überprüfen von Veränderungen können in verschiedenen Ausführungsbeispielen dem Nutzer einen Zusatznutzen einer individualisierbaren Behandlung bieten.

Es wird zum Ermitteln des Hautfeuchtigkeitsgehalts der Haut (z.B. insbesondere der Epidermis und Dermis) ein Sensor verwendet , welcher einen Gehalt an Wasser in der Haut detektiert (genauer kann ein Sensor-Messwert erfasst und daraus der Hautfeuchtigkeitsgehalt ermittelt werden). Dafür kann eine spezielle Vorrichtung genutzt werden, welche an ein Smartphone, ein Tablet oder eine andere Datenverarbeitungsvorrichtung (z.B. einen Computer) anschließbar oder darin integriert sein kann.

In verschiedenen Ausführungsbeispielen kann der Sensor zum Ermitteln der Hautfeuchtigkeit (auch als Hautfeuchtigkeitsmessvorrichtung bezeichnet) für die Messung z.B. eine Impedanz, eine Dielektrizitätskonstante, einen elektrischen Leitwert oder eine Wärmeleitfähigkeit der Haut nutzen. Dabei kann ein Messprinzip der Hautfeuchtigkeitsmessvorrichtung im Prinzip bekannt sein, z.B. kann ein Corneometer genutzt werden, um die Dielektrizitätskonstante der Haut (steigend mit größerem Wassergehalt) zu ermitteln, oder z.B. eine so genannte TTT-Vorrichtung (wobei TTT für "Transient Thermal Transfer", auf Deutsch: transiente Wärmeübertragung steht) kann genutzt werden, um die Wärmeleitfähigkeit der Haut (ebenfalls steigend mit größerem Wassergehalt) zu ermitteln. In verschiedenen Ausführungsbeispielen kann die Hautfeuchtigkeitsmessvorrichtung einen Sensorkontaktbereich aufweisen, bei welchem es nötig sein kann, dass er mit der Haut, deren Feuchtigkeitsgehalt zu ermitteln ist, in Kontakt gebracht wird.

In verschiedenen Ausführungsbeispielen kann die Hautfeuchtigkeitsmessvorrichtung (der Sensor) an einem Träger angebracht sein. Der Träger kann in verschiedenen Ausführungsbeispielen als Pflaster gestaltet sein, d.h. als flacher flexibler (z.B. biegsamer) Träger, der auf einer Seite mit einem Haftmittel versehen ist, mit welchem das Pflaster an der Haut des Nutzers befestigbar sein kann. Damit kann eine kontinuierliche Messung und/oder eine wiederholte Messung über einen längeren Zeitraum hinweg (z.B. mehrmals innerhalb von Minuten, Stunden, Tagen oder Wochen) ermöglicht sein.

Aus dem Messwert (auch als Messsignal oder Signal bezeichnet) wird in verschiedenen Ausführungsbeispielen, z.B. mittels einer elektronischen Schaltkreisvorrichtung, der Hautfeuchtigkeitsgehalt ermittelt.

In verschiedenen Ausführungsbeispielen kann mittels einer App oder einer sonstigen Software der detektierte Hautfeuchtigkeitsgehalt ermittelt und in Form eines Wertes (z.B. mit willkürlichen Einheiten), als graphische Darstellung und/oder akustische Mitteilung bereitgestellt werden.

In verschiedenen Ausführungsbeispielen kann, auch wenn der Träger nicht als Pflaster gebildet ist, der Hautfeuchtigkeitsgehalts über einen längeren Zeitraum (z.B. Minuten, Stunden, Tage o.ä.) aufgezeichnet werden.

In verschiedenen Ausführungsbeispielen kann anhand des Hautfeuchtigkeitsgehalts eine Folge einer Dysbalance beschrieben werden.

In verschiedenen Ausführungsbeispielen kann anhand des Hautfeuchtigkeitsgehalts eine individualisierte kosmetische Behandlung zur Optimierung des Zustands empfohlen werden.

Die Vorrichtung kann in verschiedenen Ausführungsbeispielen günstig und klein (z.B. tragbar) ausgebildet sein. Die Vorrichtung kann an einem (lebenden) Nutzer angewendet werden, beispielsweise bei dem Nutzer zu Hause, an einem Verkaufspunkt für Kosmetika, bei einem Hautarzt und/oder in einem Kosmetikstudio. Die Vorrichtung kann in verschiedenen Ausführungsbeispielen ein Smartphone, ein Tablet, ein iPad oder eine ähnliche elektronische Schaltkreisvorrichtung eine Datenverarbeitungsvorrichtung und/oder eine Datenübertragungsvorrichtung) aufweisen, und eine integrierte Vorrichtung oder eine Zusatzvorrichtung, die beispielsweise an der elektronischen Schaltkreisvorrichtung anbringbar und/oder damit verbindbar (z.B. mittels einer Datenverbindung verbindbar) sein kann. Die integrierte Vorrichtung bzw. die Zusatzvorrichtung (ggf. sogar die gesamte Vorrichtung) kann eine Größe aufweisen, die es ermöglicht, sie problemlos in einer Hand- oder Hosentasche unterzubringen, beispielsweise mit einer Fläche von weniger als 36 cm² und mit einer Dicke von weniger als 2 cm.

Das mittels der Hautfeuchtigkeitsmessvorrichtung ermittelte Signal wird mit Referenzsignalen für bekannte Hautfeuchtigkeitsgehalte verglichen und anhand dessen ein Hautfeuchtigkeitsgehalt des Nutzers ermittelt. Die Referenzsignale mit zugeordneten Hautfeuchtigkeitsgehalten sind in einer Datenbank in einer Prozessor-Cloud-Architektur gespeichert, wobei das Vergleichen beispielsweise derart erfolgen kann, dass Datenbankeinträge für das Referenzsignal direkt mit dem Signal verglichen werden, oder beispielsweise derart, dass das Signal mittels einer anhand von Referenzsignalen und Referenzhautfeuchtigkeitsgehalten ermittelten Beziehung zu einem Hautfeuchtigkeitsgehalt des Nutzers umgerechnet wird. Die Referenzdaten (Referenzsignale, zugeordnete Referenzhautfeuchtigkeitsgehalte) können in verschiedenen Ausführungsbeispielen vorab ermittelt worden sein, beispielsweise bei Laborversuchen, wobei die Referenzhautfeuchtigkeitsgehalte z.B. auf herkömmliche (z.B. aufwändige) Weise ermittelt worden sein können. Bei einem Speichern der Datenbank in der Prozessor-Cloud-Architektur kann in verschiedenen Ausführungsbeispielen ferner die Datenbank aktualisiert werden basierend auf neuen Zuordnungen des Hautfeuchtigkeitsgehalts zum erfassten Signal von einer Mehrzahl von weiteren Nutzern aufweisen. Das Aktualisieren kann z.B. in regelmäßigen, z.B. vorbestimmten Zeitabständen, und/oder beispielsweise immer dann, wenn einer der weiteren Nutzer eine der neuen Zuordnungen bereitstellt, erfolgen.

In verschiedenen Ausführungsbeispielen kann das Vergleichen des Signals mit dem Referenzsignal und/oder das Ermitteln des Hautfeuchtigkeitsgehalts daraus mittels der elektronischen Schaltkreisvorrichtung (z.B. direkt) erfolgen, beispielsweise mittels einer App oder eines sonstigen Softwareprogramms, welches von der elektronischen Schaltkreisvorrichtung (z.B. einem Smartphone, einem Tablet, einem iPad oder ähnlichem) betrieben wird.

In verschiedenen Ausführungsbeispielen kann das Vergleichen des Signals mit dem Referenzsignal und/oder das Ermitteln des Hautfeuchtigkeitsgehalts daraus indirekt mittels der elektronischen Schaltkreisvorrichtung erfolgen, beispielsweise indem die elektronische Schaltkreisvorrichtung eine (z.B. kontaktlose) Datenkommunikationsverbindung bereitstellt und mittels der Datenkommunikationsverbindung das Signal und/oder das Ergebnis des Vergleichs des Signals mit dem Referenzsignal einer externen Datenverarbeitungsvorrichtung (z.B. der Prozessor-Cloud-Architektur) bereitstellt und von der externen Datenverarbeitungsvorrichtung den mittels des Signals und/oder des Ergebnisses des Vergleichs ermittelten Hautfeuchtigkeitsgehalt empfängt.

Die Hautfeuchtigkeitsmessvorrichtung weist mindestens einen Sensorkontaktbereich auf, welcher dafür vorgesehen ist, für das Messen der Hautfeuchtigkeit mit der Haut (z.B. einem Hautbereich des Nutzers) in Kontakt gebracht zu werden. Im Sensorkontaktbereich können beispielsweise Elektroden eines Kondensators, Elektroden zum Anlegen einer Wechselspannung oder Wärmesonden zum Einbringen einer Wärmemenge in die Haut und zum Messen einer Temperaturerhöhung der Haut.

In verschiedenen Ausführungsbeispielen, insbesondere wenn vorgesehen ist, die Vorrichtung zum Ermitteln des Hautfeuchtigkeitsgehalts für einen längeren Zeitraum (z.B. einen Zeitraum, der länger ist, als für eine Einzelmessung erforderlich, z.B. länger als etwa eine Sekunde, also z.B. mehrere Sekunden, eine oder mehrere Minuten, oder sogar Stunden oder Tage) auf der Haut des Nutzers zu belassen, z.B. für wiederholte Messungen oder eine kontinuierliche Messung des Hautfeuchtigkeitsgehalts, kann eine Fläche des Sensorkontaktbereichs minimiert sein, beispielsweise so klein gestaltet sein, wie es bei der verwendeten Art von Hautfeuchtigkeitsmessvorrichtung möglich ist. In verschiedenen Ausführungsbeispielen kann der Sensorkontaktbereich eine Fläche von maximal etwa 1 mm² aufweisen, z.B. maximal 0,5 mm² oder weniger.

In verschiedenen Ausführungsbeispielen können der Träger und/oder eine sonstige Umgebung des Sensorkontaktbereichs derart gestaltet sein, dass ein sich Ansammeln von Feuchtigkeit, die von der Haut abgegeben wird, zwischen dem Sensorkontaktbereich und der Haut vermieden oder zumindest gemindert wird. In verschiedenen Ausführungsbeispielen kann der Träger atmungsaktiv gestaltet sein (z.B. als atmungsaktives Pflaster), beispielsweise mittels Öffnungen, welche sich von der Haut und/oder von oberhalb des Sensors durch den Träger bis außerhalb der Vorrichtung zum Ermitteln der Hautfeuchtigkeit erstrecken, wobei die Öffnungen durchlässig sein können für Dampf (z.B. Wasserdampf) und/oder für Flüssigkeiten (z.B. Wasser, Schweiß). Benachbart zu dem Sensorkontaktbereich ist ein Feuchtigkeit aufnehmendes Material angeordnet, beispielsweise derart, dass der vom Sensorkontaktbereich kontaktierte Hautbereich und ein benachbarter Hautbereich, welcher von dem Feuchtigkeit aufnehmenden Material kontaktiert wird, unmittelbar aneinander angrenzen. Das Feuchtigkeit aufnehmende Material kann z.B. den Sensorkontaktbereich umgebend angeordnet sein.

In verschiedenen Ausführungsbeispielen kann die Gestaltung, die das Ansammeln von Feuchtigkeit zwischen dem Sensorkontaktbereich und der Haut mindert oder vermeidet, es ermöglichen, wiederholt über einen längeren Zeitraum und/oder kontinuierlich den Hautfeuchtigkeitsgehalt in der Haut zu ermitteln, ohne dass die Messergebnisse bereits nach kurzer Zeit (z.B. nach mehreren Sekunden oder wenigen Minuten) durch von der Haut abgegebene und dann auf der Haut als Flüssigkeitsfilm aufliegende Feuchtigkeit verfälscht wird.

Zum Verbessern eines Signal-zu-Rauschen-Verhältnisses ist ein Gesamt-Sensorkontaktbereich dadurch vergrößert, dass eine Mehrzahl von zueinander beabstandeten Sensorkontaktbereichen bereitgestellt ist. Mittels jedes der Sensorkontaktbereiche kann ein Signal bereitgestellt werden, wobei in verschiedenen Ausführungsbeispielen aus der Mehrzahl von Signalen ein Mittelwert gebildet werden kann. In einem Abstandsbereich zwischen den Sensorkontaktbereichen ist das Feuchtigkeit aufnehmende Material angeordnet

Dem Nutzer kann der ermittelte Hautfeuchtigkeitsgehalt in verschiedenen Ausführungsbeispielen auf beliebige, z.B. vom Nutzer wählbare, Art bereitgestellt werden, z.B. als ein Zahlenwert (in willkürlichen oder physikalischen Einheiten, z.B. als Gewichtsanteil, z.B. in Prozent), eine graphische Darstellung (beispielsweise einer Darstellung des ermittelten Werts in im Verhältnis zu einem gesamten Wertebereich von sehr feuchtigkeitsarm über normal bis hin zu sehr feuchtigkeitsreich), eine akustische Mitteilung, oder ähnliches.

Insbesondere in einem Fall, in welchem der Hautfeuchtigkeitsgehalt für eine Mehrzahl von Hautbereichen ermittelt wird, kann das Bereitstellen des Hautfeuchtigkeitsgehalts an den Nutzer eine graphische Darstellung aufweisen, beispielsweise eine Anzeige, z.B. mittels einer Anzeigevorrichtung (z.B. eines Displays eines Smartphones, Tablets, iPads oder Smart Mirrors). In der graphischen Darstellung kann der ermittelte Hautfeuchtigkeitsgehalt der Mehrzahl von Bereichen mit einer Codierung anhand des Hautfeuchtigkeitsgehalts in einer Darstellung des Nutzers (z.B. einer schematischen Darstellung oder auf einem Foto des Nutzers) angezeigt werden. Beispielsweise können in einer schematischen Darstellung eines Gesichts des Nutzers Bereiche unterschiedlichen Hautfeuchtigkeitsgehalts mit unterschiedlichen Farben und/oder Mustern dargestellt werden, z.B. Bereiche mit normalem Hautfeuchtigkeitsgehalt grün, mit niedrigem Hautfeuchtigkeitsgehalt rot und mit hohem Hautfeuchtigkeitsgehalt blau oder ähnliches. In einem anderen Beispiel können einem Foto des Nutzers, z.B. einem digitalen Foto, welches das Gesicht des Nutzers zeigt, unterschiedliche Muster überlagert sein, z.B. ein Punktmuster für Bereiche mit hohem Hautfeuchtigkeitsgehalt und ein Linienmuster für Bereiche mit niedrigem Hautfeuchtigkeitsgehalt, wobei Bereiche normaler Haut ungemustert verbleiben können, oder ähnliches. Alternativ kann die Echtzeit-Darstellung eines Körpers oder eines Körperbereichs (z.B. des Gesichts) auf/in einem Smart Mirror zur Darstellung der ermittelten Hautfeuchtigkeitsgehalte verwendet werden.

Beim graphischen Darstellen des Hautfeuchtigkeitsgehalts des mindestens einen Hautbereichs, z.B. der Mehrzahl der Hautbereiche, kann in verschiedenen Ausführungsbeispielen nur für den Bereich/die Bereiche, für den/die der Hautfeuchtigkeitsgehalt ermittelt wurde, der Hautfeuchtigkeitsgehalt dargestellt werden. In verschiedenen Ausführungsbeispielen kann ein Bereich, für den ein ermittelter Hautfeuchtigkeitsgehalt angezeigt wird, über den Hautbereich hinaus, für den der Hautfeuchtigkeitsgehalt ermittelt wurde, extrapoliert werden, beispielsweise unter Einbeziehung typischer Hautfeuchtigkeitsverteilungsmuster.

Dabei kann in verschiedenen Ausführungsbeispielen eine Information darüber, wo der Bereich/die Bereiche ist/sind, der elektronischen Schaltkreisvorrichtung bereitgestellt werden, beispielsweise mittels des Nutzers, z.B. mittels einer Eingabevorrichtung (z.B. Antippen eines berührungsempfindlichen Displays, auf welchem eine schematische oder fotografische Darstellung des Nutzers angezeigt wird, mittels Texteingabe über eine Tastatur oder jede andere geeignete Eingabemöglichkeit).

In verschiedenen Ausführungsbeispielen kann die Anzeigevorrichtung Teil der Vorrichtung sein, z.B. ein Display eines Smartphones, Tablets oder iPads. In verschiedenen Ausführungsbeispielen kann die Anzeigevorrichtung mit der Vorrichtung gekoppelt sein, beispielsweise mittels einer (z.B. kabellosen) Datenverbindung.

Zusätzlich oder alternativ kann in verschiedenen Ausführungsbeispielen dem ermittelten Hautfeuchtigkeitsgehalt ein Qualitätswert zugeordnet werden, mit einem hohen Qualitätswert für normale/gesunde Haut und einem niedrigen Qualitätswert für zu feuchtigkeitsarme/feuchtigkeitsreiche Haut, wobei typischerweise eher zu feuchtigkeitsarme als zu feuchtigkeitsreiche Haut problematisch ist.

In verschiedenen Ausführungsbeispielen soll es einem Nutzer ermöglicht werden, gezielt kosmetische Produkte zu finden und/oder Pflegeratschläge zu erhalten, die auf individuelle Bedürfnisse der Haut des Nutzers abgestimmt sein können.

In verschiedenen Ausführungsbeispielen kann dem Nutzer, basierend auf dem ermittelten Hautfeuchtigkeitsgehalt, mindestens ein Kosmetikprodukt empfohlen werden. Das Kosmetikprodukt kann geeignet sein, einen Hautfeuchtigkeitsgehalt des Nutzers zu erhalten oder zu verbessern (z.B. einen Hautfeuchtigkeitsgehalt zu normalisieren oder in einem Normalzustand zu stabilisieren). In verschiedenen Ausführungsbeispielen kann das Kosmetikprodukt eine Creme, eine Lotion, eine Salbe, ein Öl, eine Emulsion, ein Gel, eine Seife, eine Maske, eine Ampulle mit einem Zusatzpflegeprodukt, ein Gesichtswasser, ein Serum, einen Spray oder ähnliches aufweisen.

Die Normalisierung oder Stabilisierung eines Hautfeuchtigkeitsgehaltes durch ein Kosmetikprodukt kann mit Hilfe von feuchtigkeitsspendende und/oder feuchtigkeitbindende Inhaltsstoffe erfolgen. Feuchtigkeitsspendende und/oder feuchtigkeitbindende Inhaltsstoffe umfassen insbesondere Aloe Vera, Pflanzenöle wie Hanföl oder Nachtkerzen-Öl, ungesättigte Fettsäuren, Harnstoff, Glycerin und/oder Dexpanthenol.

In verschiedenen Ausführungsbeispielen können die Produktempfehlungen und/oder Pflegehinweise beispielsweise mittels einer Software, z.B. einer App, bereitgestellt werden. Beispielsweise kann dem Nutzer empfohlen werden, ein kosmetisches Pflegeprodukt, welches feuchtigkeitsspendend wirkt, auf dem mindestens einen Hautbereich mit niedrigem Hautfeuchtigkeitsgehalt anzuwenden, und/oder viel (z.B. Wasser) zu trinken.

In verschiedenen Ausführungsbeispielen kann die Software/App, welche den Hautfeuchtigkeitsgehalt ermittelt, dieselbe sein, die die Produktempfehlung und/oder die Pflegehinweise ermittelt. In verschiedenen Ausführungsbeispielen können unterschiedliche Softwareprogramme/Apps verwendet werden für einen Teil der verschiedenen Vorgänge oder alle verschiedenen Vorgänge (Ermitteln des Hautfeuchtigkeitsgehalts, Ermitteln einer Produktempfehlung, Ermitteln eines Pflegehinweises).

In verschiedenen Ausführungsbeispielen kann ein Behandlungserfolg bei einer kosmetischen Behandlung, welche ein positives Beeinflussen des ermittelten Hautfeuchtigkeitsgehalts zum Ziel haben kann, überwacht werden. In verschiedenen Ausführungsbeispielen kann die Software/App eine Kontrolle und/oder Nachverfolgung der Ergebnisse mittels einer Darstellung (z.B. einer graphischen Darstellung) der Messergebnisse im Verlauf der Zeit ermöglichen.

In verschiedenen Ausführungsbeispielen können beim Ermitteln der Produkt- und Pflegeempfehlungen ferner Informationen hinsichtlich eines generellen Gesundheitszustands, eines Hautzustands, Ernährungsgewohnheiten und weiterer Verhaltensweisen des Nutzers (z.B. tägliche Aufenthaltsdauer im Freien/in der Sonne/im Wasser, Rauchgewohnheiten, Sportgewohnheiten, usw.) verwendet werden, z.B. mittels der Software/App. Diese Informationen können in verschiedenen Ausführungsbeispielen mittels der Software/App vom Nutzer erfragt werden.

In verschiedenen Ausführungsbeispielen können zum Beurteilen einer Eignung eines Pflegeprodukts und/oder eines Pflegehinweises zum Pflegen einer Haut mit einem gegebenen Hautfeuchtigkeitsgehalt Literaturdaten zu Grunde gelegt werden.

In verschiedenen Ausführungsbeispielen kann jedem Hautfeuchtigkeitsgehalt einer Mehrzahl von Hautfeuchtigkeitsgehalten ein Qualitätswert zugeordnet sein oder werden. Ein Pflegeprodukt und/oder ein Pflegehinweis kann für einen Hautfeuchtigkeitsgehalt als geeignet bewertet werden, wenn zu erwarten ist, z.B. aufgrund von Literaturdaten, Versuchsergebnissen oder Erfahrungswerten (welche beispielsweise fortlaufend, z.B. auch während einer Nutzung der Software/der App durch den Nutzer, mit neuen Erfahrungswerten anderer Nutzer aktualisiert werden können, siehe unten), dass bei einer (z.B. regelmäßigen) Anwendung des Pflegeprodukts und/oder des Pflegehinweises der Hautfeuchtigkeitsgehalt des Nutzers aufrechterhalten wird oder sich zu einem Hautfeuchtigkeitsgehalt mit einem höheren (d.h. besseren) Qualitätswert ändert.

In verschiedenen Ausführungsbeispielen kann eine Beurteilung einer Eignung eines Pflegeproduktes, einen Hautfeuchtigkeitsgehalt zu verbessern, bestätigt oder abgeändert werden mittels eines Aufnehmens von Erfahrungswerten weiterer Nutzer mit demselben oder einem ähnlichen Hautfeuchtigkeitsgehalt, beispielsweise von Erfahrungswerten hinsichtlich eines Behandlungserfolgs. Vorzugsweise weisen die weiteren Nutzer neben demselben oder ähnlichen Hautfeuchtigkeitsgehalt auch dasselbe oder ein ähnliches Profil in Bezug auf Alter, Geschlecht, generellen Gesundheitszustand, generellen Hautzustand abgesehen vom Hautfeuchtigkeitsgehalt und/oder Verhaltensweisen wie beispielsweise Rauchen, Ernährung und/oder Sport, auf. Damit kann ermöglicht sein, dass der Nutzer immer eine optimale Empfehlung erhält.

In verschiedenen Ausführungsbeispielen können eine Kontrolle und eine Nachverfolgung einer Wirksamkeit einer kosmetischen Behandlung auf objektive und standardisierte Art und Weise ermöglicht sein. Die kosmetische Behandlung kann in verschiedenen Ausführungsbeispielen das Ziel haben, einen Hautfeuchtigkeitsgehalt zu normalisieren.

In verschiedenen Ausführungsbeispielen kann eine Wirksamkeit einer (z.B. kosmetischen) Behandlung besser nachvollzogen werden und dadurch eine Auswahl eines individuell geeigneten Produkts vereinfacht sein oder werden.

In verschiedenen Ausführungsbeispielen kann eine Motivation eines Nutzers erhöht werden, eine kosmetische Behandlung längerfristig durchzuführen, beispielsweise mittels eines Vergleichs mit anderen Nutzern, z.B. mittels von den anderen Nutzern bereitgestellten Informationen über Behandlungserfolge.

In verschiedenen Ausführungsbeispielen wird ein Verfahren zum Ermitteln eines Hautfeuchtigkeitsgehalts bereitgestellt (nicht Teil der beanspruchten Erfindung).

Das Verfahren kann für mindestens einen Hautbereich eines Nutzers ein Kontaktieren des mindestens einen Hautbereichs mittels mindestens eines Sensorkontaktbereichs einer Hautfeuchtigkeitsmessvorrichtung aufweisen, ein Erfassen eines mittels der Hautfeuchtigkeitsmessvorrichtung bereitgestellten, von einer Hautfeuchtigkeit im Hautbereich abhängigen Signals und ein Zuordnen eines Hautfeuchtigkeitsgehalts zum erfassten Signal mittels Vergleichens des erfassten Signals mit Referenzsignalen für bekannte Hautfeuchtigkeitsgehalte, wobei das Zuordnen des Hautfeuchtigkeitsgehalts zum erfassten Signal mittels einer in einer Prozessor-Cloud-Architektur gespeicherten Datenbank erfolgen kann.

In verschiedenen Ausführungsbeispielen kann das Verfahren ferner ein Aktualisieren der Datenbank basierend auf neuen Zuordnungen des Hautfeuchtigkeitsgehalts zum erfassten Signal von einer Mehrzahl von weiteren Nutzern aufweisen.

In verschiedenen Ausführungsbeispielen kann die Hautfeuchtigkeitsmessvorrichtung mindestens eine aus einer Gruppe von Hautfeuchtigkeitsmessvorrichtungen aufweisen, wobei die Gruppe eine kapazitive Hautfeuchtigkeitsmessvorrichtung, eine Impedanz-Hautfeuchtigkeitsmessvorrichtung, eine Hautfeuchtigkeitsmessvorrichtung, welche den elektrischen Leitwert der Haut nutzt, und eine Hautfeuchtigkeitsmessvorrichtung, welche eine transiente Wärmeübertragung der Haut nutzt, aufweisen kann.

In verschiedenen Ausführungsbeispielen kann nach dem Kontaktieren des mindestens einen Hautbereichs mittels des mindestens einen Sensorkontaktbereichs der Hautfeuchtigkeitsmessvorrichtung das Erfassen des Signals und das Zuordnen des Hautfeuchtigkeitsgehalts zum erfassten Signal mehrmals mit zeitlichem Abstand oder kontinuierlich ausgeführt werden.

In verschiedenen Ausführungsbeispielen kann der mindestens eine Sensorkontaktbereich eine Mehrzahl von voneinander beabstandeten Sensorkontaktbereichen aufweisen, und das Verfahren kann ferner ein Bilden eines Signal-Mittelwerts aus der Mehrzahl von Signalen der Mehrzahl von Sensorkontaktbereichen aufweisen.

In verschiedenen Ausführungsbeispielen kann der mindestens eine Hautbereich eine Mehrzahl von Hautbereichen des Nutzers aufweisen.

In verschiedenen Ausführungsbeispielen kann die Hautfeuchtigkeitsmessvorrichtung Teil einer tragbaren Vorrichtung sein.

In verschiedenen Ausführungsbeispielen kann die tragbare Vorrichtung ein Smartphone, ein Tablet oder ein iPad aufweisen oder an einem Smartphone, einem Tablet oder einem iPad anbringbar sein.

In verschiedenen Ausführungsbeispielen kann das Erfassen des Signals und/oder das Zuordnen des Hautfeuchtigkeitsgehalts zum erfassten Signal mittels einer App erfolgen.

In verschiedenen Ausführungsbeispielen wird ein Verfahren zum Ermitteln einer kosmetischen Hautbehandlungsempfehlung bereitgestellt. Das Verfahren kann ein Ermitteln eines Hautfeuchtigkeitsgehalts gemäß verschiedenen Ausführungsbeispielen und ein Ermitteln des Kosmetikprodukts und/oder einer Pflegeanweisung aufweisen basierend auf dem ermittelten Hautfeuchtigkeitsgehalt und einer in der Prozessor-Cloud-Architektur gespeicherten weiteren Datenbank, welche eine Mehrzahl von Hautfeuchtigkeitsgehalten und eine Mehrzahl von zugeordneten Kosmetikprodukten und/oder Pflegeanweisungen aufweist, wobei jedem Hautfeuchtigkeitsgehalts der Mehrzahl von Hautfeuchtigkeitsgehalten mindestens ein geeignetes Kosmetikprodukt und/oder mindestens eine Pflegeanweisung zugeordnet ist.

In verschiedenen Ausführungsbeispielen kann/können das Kosmetikprodukt und/oder die Pflegeanweisung geeignet sein für den Hautfeuchtigkeitsgehalt, wenn basierend auf in der weiteren Datenbank gespeicherten Erfahrungswerten einer Mehrzahl weiterer Nutzer mit dem Kosmetikprodukt eine Normalisierung des Hautfeuchtigkeitsgehalts zu erwarten ist.

In verschiedenen Ausführungsbeispielen kann das Ermitteln des Hautfeuchtigkeitsgehalts und/oder das Ermitteln der kosmetischen Hautbehandlungsempfehlung ein Übertragen des Signals und/oder des Hautfeuchtigkeitsgehalts an eine externe Datenverarbeitungsvorrichtung und ein Empfangen des Hautfeuchtigkeitsgehalts und/oder der kosmetischen Hautbehandlungsempfehlung aufweisen.

In verschiedenen Ausführungsbeispielen wird eine Vorrichtung zum Ermitteln eines Hautfeuchtigkeitsgehalts bereitgestellt. Die Vorrichtung kann eine Hautfeuchtigkeitsmessvorrichtung aufweisen, welche mindestens einen Sensorkontaktbereich aufweisen kann und eingerichtet sein kann, bei einem Kontaktieren mindestens eines Hautbereichs eines Nutzers mittels des mindestens einen Sensorkontaktbereichs ein von einer Hautfeuchtigkeit im Hautbereich abhängiges Signal bereitzustellen, eine elektronische Schaltkreisvorrichtung zum Erfassen des von der Hautfeuchtigkeitsmessvorrichtung bereitgestellten Signals und zum Zuordnen eines Hautfeuchtigkeitsgehalts zum erfassten Signal mittels Vergleichens des erfassten Signals mit Referenzsignalen für bekannte Hautfeuchtigkeitsgehalte, wobei das Zuordnen des Hautfeuchtigkeitsgehalts zum erfassten Signal mittels einer in einer Prozessor-Cloud-Architektur gespeicherten Datenbank erfolgt.

In verschiedenen Ausführungsbeispielen kann die Hautfeuchtigkeitsmessvorrichtung mindestens eine aufweisen aus einer Gruppe von Hautfeuchtigkeitsmessvorrichtungen, wobei die Gruppe eine kapazitive Hautfeuchtigkeitsmessvorrichtung, eine Impedanz-Hautfeuchtigkeitsmessvorrichtung, eine Hautfeuchtigkeitsmessvorrichtung, welche den elektrischen Leitwert der Haut nutzt, und eine Hautfeuchtigkeitsmessvorrichtung, welche eine transiente Wärmeübertragung der Haut nutzt, aufweisen kann.

In verschiedenen Ausführungsbeispielen kann der mindestens eine Sensorkontaktbereich eine Fläche von weniger als 1 mm² aufweisen.

In verschiedenen Ausführungsbeispielen kann die Vorrichtung ferner einen atmungsaktiven Träger aufweisen, wobei die Hautfeuchtigkeitsmessvorrichtung und die elektronische Schaltkreisvorrichtung auf dem atmungsaktiven Träger angeordnet sind.

In verschiedenen Ausführungsbeispielen kann der atmungsaktive Träger mit der Hautfeuchtigkeitsmessvorrichtung und der elektronischen Schaltkreisvorrichtung als haftendes Pflaster gestaltet sein.

In verschiedenen Ausführungsbeispielen kann die Vorrichtung ferner eine auf dem atmungsaktiven Träger angeordnete kabellose Datenaustauschvorrichtung zum Austauschen von Daten zwischen der elektronischen Schaltkreisvorrichtung und der Prozessor-Cloud-Architektur aufweisen.

Es wird eine Vorrichtung zum Ermitteln eines Hautfeuchtigkeitsgehalts bereitgestellt. Die Vorrichtung kann einen atmungsaktiven Träger, eine Hautfeuchtigkeitsmessvorrichtung, welche auf dem atmungsaktiven Träger angeordnet sein kann und mindestens einen Sensorkontaktbereich aufweisen und eingerichtet sein kann, bei einem Kontaktieren mindestens eines Hautbereichs eines Nutzers mittels des mindestens einen Sensorkontaktbereichs ein von einer Hautfeuchtigkeit im Hautbereich abhängiges Signal bereitzustellen, und eine auf dem atmungsaktiven Träger angeordnete elektronische Schaltkreisvorrichtung zum Erfassen des von der Hautfeuchtigkeitsmessvorrichtung bereitgestellten Signals und zum Zuordnen eines Hautfeuchtigkeitsgehalts zum erfassten Signal mittels Vergleichens des erfassten Signals mit Referenzsignalen für bekannte Hautfeuchtigkeitsgehalte.

In verschiedenen Ausführungsbeispielen kann der atmungsaktive Träger mit der Hautfeuchtigkeitsmessvorrichtung und der elektronischen Schaltkreisvorrichtung als haftendes Pflaster gestaltet sein.

In den Zeichnungen beziehen sich ähnliche Bezugszeichen üblicherweise auf dieselben Teile in allen unterschiedlichen Ansichten, wobei der Übersichtlichkeit wegen teilweise darauf verzichtet wird, sämtliche einander entsprechenden Teile in allen Figuren mit Bezugszeichen zu versehen. Teile derselben oder ähnlicher Art können zur Unterscheidung zusätzlich zu einem gemeinsamen Bezugszeichen mit einer nachgestellten Ziffer oder einem nachgestellten kleinen Buchstaben versehen sein. Die Zeichnungen sollen nicht notwendigerweise eine maßstabgetreue Wiedergabe darstellen, sondern die Betonung liegt vielmehr auf einem Veranschaulichen der Prinzipien der Erfindung. In der folgenden Beschreibung werden verschiedene Ausführungsformen der Erfindung mit Bezug auf die folgenden Zeichnungen beschrieben, in denen:
FIG. 1 eine Vorrichtung zum Ermitteln eines Hautfeuchtigkeitsgehalts gemäß verschiedenen Ausführungsbeispielen zeigt und ein Verwenden der Vorrichtung veranschaulicht;
FIG. 2A und FIG. 2B jeweils eine Vorrichtung zum Ermitteln eines Hautfeuchtigkeitsgehalts gemäß verschiedenen Ausführungsbeispielen zeigen;
FIG. 3A, FIG. 3B und FIG. 3C jeweils eine Vorrichtung zum Ermitteln eines Hautfeuchtigkeitsgehalts gemäß verschiedenen Ausführungsbeispielen zeigen und ein Verwenden der Vorrichtung veranschaulichen;
FIG. 4 Hautregionen zum Anwenden eines Verfahrens und einer Vorrichtung zum Ermitteln eines Hautfeuchtigkeitsgehalts gemäß verschiedenen Ausführungsbeispielen veranschaulicht;
FIG. 5 ein Flussdiagramm zum Veranschaulichen eines Verfahrens zum Ermitteln einer Hautbehandlungsempfehlung gemäß verschiedenen Ausführungsbeispielen zeigt;
FIG. 6 ein Flussdiagramm eines Verfahrens zum Ermitteln eines Hautfeuchtigkeitsgehalts gemäß verschiedenen Ausführungsbeispielen zeigt; und
FIG. 7 ein Flussdiagramm eines Verfahrens zum Ermitteln einer kosmetischen Hautbehandlungsempfehlung gemäß verschiedenen Ausführungsbeispielen zeigt.

Die folgende ausführliche Beschreibung bezieht sich auf die begleitenden Zeichnungen, die als Beispiel durch Veranschaulichung bestimmte Details und Ausführungen zeigen, in denen die Erfindung in die Praxis umgesetzt werden kann.

Das Wort "beispielhaft" wird hierin in der Bedeutung von "als ein Beispiel, ein Exemplar oder eine Veranschaulichung dienend" verwendet. Alle hierin als "beispielhaft" beschriebenen Ausführungsformen oder Ausgestaltungen sind nicht notwendigerweise als bevorzugt oder vorteilhaft anderen Ausführungsformen oder Ausgestaltungen gegenüber zu deuten.

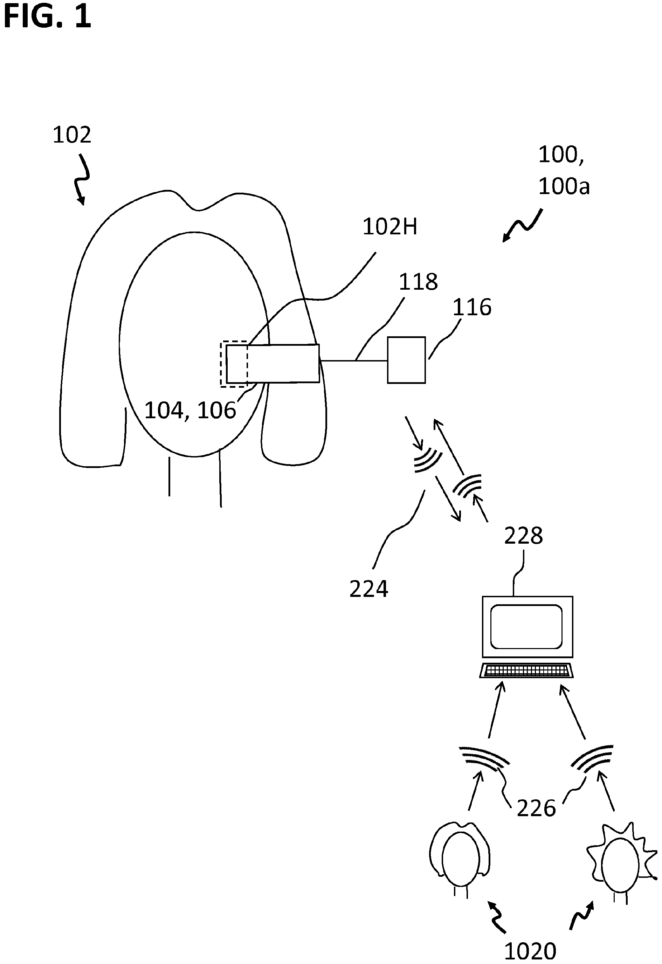

FIG. 1, FIG. 2A, FIG. 2B, FIG. 3A, FIG. 3B und FIG. 3C zeigen jeweils eine Vorrichtung 100 (100a, 100b, 100c, 100d, 100e oder 100f) zum Ermitteln eines Hautfeuchtigkeitsgehalts eines Nutzers 102 gemäß verschiedenen Ausführungsbeispielen.

Die Vorrichtung 100 zum Ermitteln eines Hautfeuchtigkeitsgehalts weist eine Hautfeuchtigkeitsmessvorrichtung 106 welche mindestens einen Sensorkontaktbereich 106K aufweist und eingerichtet ist, bei einem Kontaktieren mindestens eines Hautbereichs 102H eines Nutzers 102 mittels des mindestens einen Sensorkontaktbereichs 102K ein von einer Hautfeuchtigkeit im Hautbereich 102H abhängiges Signal bereitzustellen.

In verschiedenen Ausführungsbeispielen kann die Hautfeuchtigkeitsmessvorrichtung 106, wie oben beschrieben, mindestens eine aufweisen aus einer Gruppe von Hautfeuchtigkeitsmessvorrichtungen 106, wobei die Gruppe eine kapazitive Hautfeuchtigkeitsmessvorrichtung (auch als Corneometer^{®} bezeichnet), eine Impedanz-Hautfeuchtigkeitsmessvorrichtung, eine Hautfeuchtigkeitsmessvorrichtung, welche den elektrischen Leitwert der Haut nutzt, und eine Hautfeuchtigkeitsmessvorrichtung, welche eine transiente Wärmeübertragung der Haut nutzt (auch als TTT-Messgerät bezeichnet), aufweisen kann. Entsprechend kann der Sensorkontaktbereich 102K in verschiedenen Ausführungsbeispielen Elektroden eines Kondensators oder zum Anlegen einer Wechselspannung oder Sonden zum Einbringen von Wärme in die Haut und zum Messen einer Temperaturänderung. Dabei kann ein prinzipieller Aufbau und Betrieb, abgesehen von hierin beschriebenen Unterschieden, im Wesentlichen demjenigen einer entsprechenden herkömmlichen Hautfeuchtigkeitsmessvorrichtung 106 entsprechen.

In verschiedenen Ausführungsbeispielen kann der mindestens eine Sensorkontaktbereich eine typische Fläche aufweisen, z.B. größer als etwa 1 mm², beispielsweise in einem Bereich von etwa 1 mm² bis etwa 1 cm² oder größer.

In verschiedenen Ausführungsbeispielen kann der mindestens eine Sensorkontaktbereich eine Fläche von weniger als 1 mm² aufweisen. Ein sehr kleiner Sensorkontaktbereich kann dazu beitragen, ein Entstehen eines Schweißfilms zwischen der Haut und dem Sensorkontaktbereich der Hautfeuchtigkeitsmessvorrichtung 106 zu verringern oder zu verhindern. Dies kann insbesondere dann genutzt werden, wenn die Vorrichtung zum Ermitteln der Hautfeuchtigkeit 100 dafür vorgesehen ist, für längere Zeit, wie z.B. an anderer Stelle beschrieben, zu verbleiben für eine wiederholte oder kontinuierliche Messung der Hautfeuchtigkeit.

In verschiedenen Ausführungsbeispielen kann ein Verhindern oder Verringern des Ansammelns von Feuchtigkeit (Schweiß) zwischen dem Sensorkontaktbereich 106K und der Haut es ermöglichen, dass der Feuchtigkeitsgehalt der Haut (z.B. der Epidermis und der Dermis) gemessen wird, und nicht die Feuchtigkeit auf der Hautoberfläche, d.h. fehlerhaft erhöhte Messungen können vermieden werden.

In verschiedenen Ausführungsbeispielen kann mittels eines kontinuierlichen Ermittelns der Hautfeuchte eine Kontrolle und Nachverfolgung der (Entwicklung der) Hautfeuchte bereitgestellt werden, z.B. mittels Bereitstellens der Messergebnisse im Verlauf der Zeit, z.B. mittels der Software/App, welche die Information beispielsweise auf einem Display darstellen kann (oder eine der anderen oben beschriebenen Möglichkeiten, dem Nutzer 102 die Messergebnisse bereitzustellen, nutzen kann). Ferner kann in verschiedenen Ausführungsbeispielen die Vorrichtung 100 eingerichtet sein, den Nutzer bei einer drohenden Unterversorgung der Haut mit Feuchtigkeit zu warnen und zu mindestens einer Gegenmaßnahme zu raten, z.B. einem Trinken von Wasser und/oder einem Anwenden einer Hautpflege.

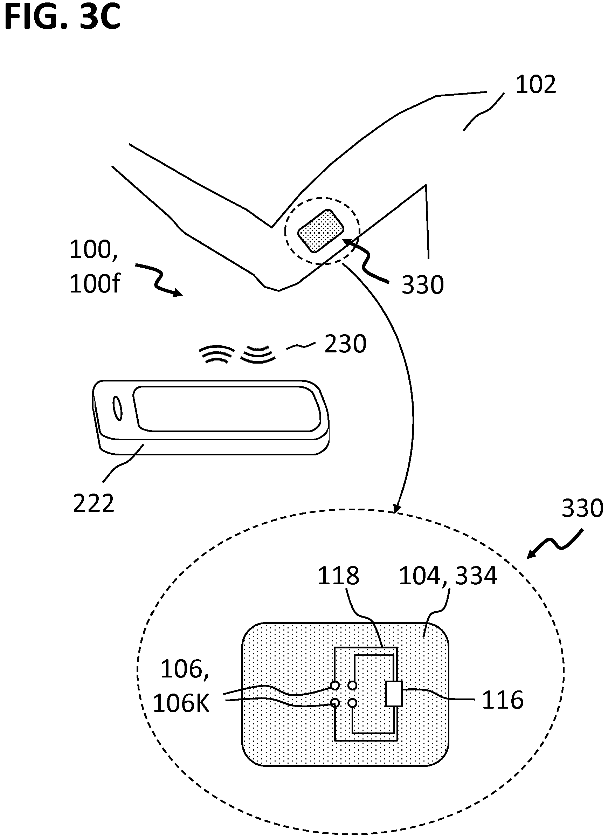

Zum Verbessern eines Signal-zu-Rauschen-Verhältnisses ist ein Gesamt-Sensorkontaktbereich (welcher als eine Summe der Sensorkontaktbereiche 106K gebildet sein kann) dadurch vergrößert, dass eine Mehrzahl von zueinander beabstandeten Sensorkontaktbereichen 106K bereitgestellt ist, wie dies in FIG. 3C dargestellt ist. Beispielhaft weist die Vorrichtung 100f vier Sensorkontaktbereiche 106K auf, welche in einer unteren, vergrößerten Abbildung einen als Pflaster gebildeten Träger 104 mit den darauf angeordneten Sensorkontaktbereichen 106 als Draufsicht von unten darstellt, d.h. von einer Seite, die während einer Verwendung der Vorrichtung 100fder Haut des Nutzers 102 zugewandt ist.

Mittels jedes der Sensorkontaktbereiche 106K wird ein Signal bereitgestellt, wobei in verschiedenen Ausführungsbeispielen aus der Mehrzahl von Signalen ein Mittelwert gebildet werden kann. In einem Abstandsbereich zwischen den Sensorkontaktbereichen 106K ist ein Feuchtigkeit aufnehmendes Material angeordnet.

In verschiedenen Ausführungsbeispielen kann die Vorrichtung 100 einen Träger 104 aufweisen, auf und/oder in welchem die Hautfeuchtigkeitsmessvorrichtung 106 angeordnet, z.B. befestigt sein kann. In verschiedenen Ausführungsbeispielen kann der Träger 104 flexibel gestaltet sein, beispielsweise als biegsamer Film, z.B. aus einem Kunststoffmaterial.

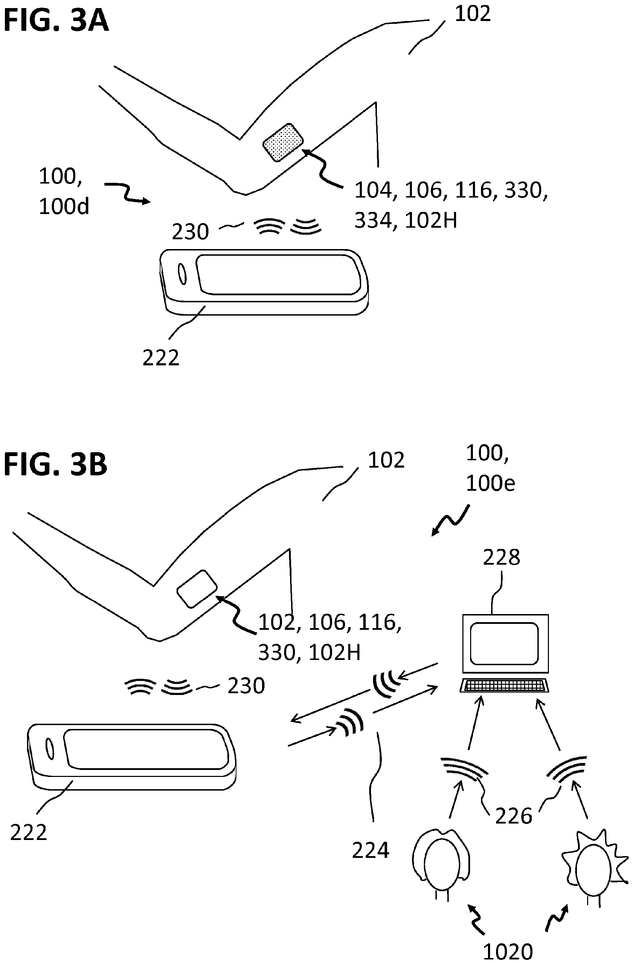

In verschiedenen Ausführungsbeispielen kann der Träger 104 atmungsaktiv gestaltet sein, beispielsweise, wie in FIG. 3A und FIG. 3C dargestellt, mit Öffnungen 334 versehen sein, welche sich, bei Anordnung des Trägers 104 derart, dass die Sensorkontaktbereiche 106K mit der Haut in Kontakt sind, von der Haut und/oder von oberhalb des Sensors106 durch den Träger 104 bis außerhalb des Trägers 104 erstrecken, wobei die Öffnungen durchlässig sein können für Dampf (z.B. Wasserdampf) und/oder für Flüssigkeiten (z.B. Wasser, Schweiß).

In FIG. 3A bis FIG. 3C ist der Träger 104 beispielhaft als (flexibles) Pflaster gebildet, welches dafür vorgesehen ist, mittels eines Haftmittels an der Haut des Nutzers 102 befestigt zu werden, wobei die Pflaster 104 in FIG. 3A und FIG. 3C atmungsaktiv gebildet sind.

Die Vorrichtung 100 zum Ermitteln des Hautfeuchtigkeitsgrads weist eine elektronische Schaltkreisvorrichtung 116 zum Erfassen des von der Hautfeuchtigkeitsmessvorrichtung 106 bereitgestellten Signals und zum Zuordnen eines Hautfeuchtigkeitsgehalts zum erfassten Signal mittels Vergleichens des erfassten Signals mit Referenzsignalen für bekannte Hautfeuchtigkeitsgehalte auf.

Die Referenzsignale (bzw. allgemein Referenzdaten) können in verschiedenen Ausführungsbeispielen, z.B. wie oben beschrieben, in Laborversuchen ermittelt und ggf. mittels zusätzlichen Daten weiterer Nutzer 1020, ggf. auch weiteren Daten zusätzlicher Laborversuche, aktualisiert worden sein bzw. werden.

Die Datenbank ist in einer externen Datenverarbeitungsvorrichtung 228, in einer Prozessor-Cloud-Architektur 228 gespeichert.

Das Zuordnen des Hautfeuchtigkeitsgehalts zum erfassten Signal mittels der in der externen Datenverarbeitungsvorrichtung 228, z.B. der Prozessor-Cloud-Architektur 228, gespeicherten Datenbank kann direkt mittels der elektronischen Schaltkreisvorrichtung 116 oder indirekt, z.B. von der elektronischen Schaltkreisvorrichtung 116 initiiert erfolgen, z.B. mittels der externen Datenverarbeitungsvorrichtung 228, z.B. der Cloud 228.

Die elektronische Schaltkreisvorrichtung 116 kann in verschiedenen Ausführungsbeispielen einen Prozessor und einen Speicher aufweisen. Die elektronische Schaltkreisvorrichtung 116 kann in verschiedenen Ausführungsbeispielen mit der Hautfeuchtigkeitsmessvorrichtung 106 gekoppelt sein, beispielsweise derart, dass ein Erfassen eines Signals, welches von der Hautfeuchtigkeitsmessvorrichtung 106 bereitgestellt wird, ermöglicht ist, beispielsweise mittels einer Datenverbindung 118. Die Datenverbindung 118 kann beispielsweise ein Datenkabel, eine interne leitende Verbindung und/oder eine kabellose Übertragung, z.B. mittels WLAN, Thread, ZigBee oder Bluetooth, aufweisen. Die elektronische Schaltkreisvorrichtung 116 kann beispielsweise ein Smartphone, ein iPad, ein Tablet, ein Laptop oder Ähnliches sein.

In verschiedenen Ausführungsbeispielen kann die elektronische Schaltkreisvorrichtung 116 auf und/oder in dem (z.B. atmungsaktiven) Träger 104 angeordnet sein.

In verschiedenen Ausführungsbeispielen kann der (z.B. atmungsaktive) Träger 104 mit der Hautfeuchtigkeitsmessvorrichtung 106 und der elektronischen Schaltkreisvorrichtung 116 als haftendes Pflaster gestaltet sein, wie in FIG. 3A bis FIG. 3C dargestellt. In FIG. 3A und FIG. 3C kann das Pflaster mittels der Öffnungen 334 atmungsaktiv gestaltet sein.

In verschiedenen Ausführungsbeispielen, wie in FIG. 3B beispielhaft dargestellt, kann das Pflaster so gestaltet sein, dass um den mindestens einen Sensorkontaktbereich 106K herum feuchtigkeitaufnehmendes Material angeordnet ist (in FIG. 3B durch das Pflaster verdeckt), so dass das Pflaster beispielsweise geschützt sein kann vor einem Eindringen von Wasser von außen, und dennoch ein Entstehen eines Flüssigkeitsfilms zwischen dem Sensorkontaktbereich 106K und der Haut vermindert oder verhindert werden kann. Das Flüssigkeit aufnehmende Material kann auch in anderen Ausführungsbeispielen, z.B. den Vorrichtungen 100a, 100b, 100c, 100d und 100f angewendet werden.

In verschiedenen Ausführungsbeispielen kann die Vorrichtung 100 ferner eine kabellose Datenaustauschvorrichtung zum Austauschen von Daten zwischen der elektronischen Schaltkreisvorrichtung 116 und der Prozessor-Cloud-Architektur aufweisen. Diese ist in den Figuren nicht gesondert dargestellt, weil sie in verschiedenen Ausführungsbeispielen als Teil der elektronischen Schaltkreisvorrichtung 116 gebildet sein kann. In verschiedenen Ausführungsbeispielen kann die kabellose Datenaustauschvorrichtung alternativ als eigenständige Datenaustauschvorrichtung gebildet sein, welche mittels einer Datenverbindung mit der elektronischen Schaltkreisvorrichtung 116 verbunden ist.

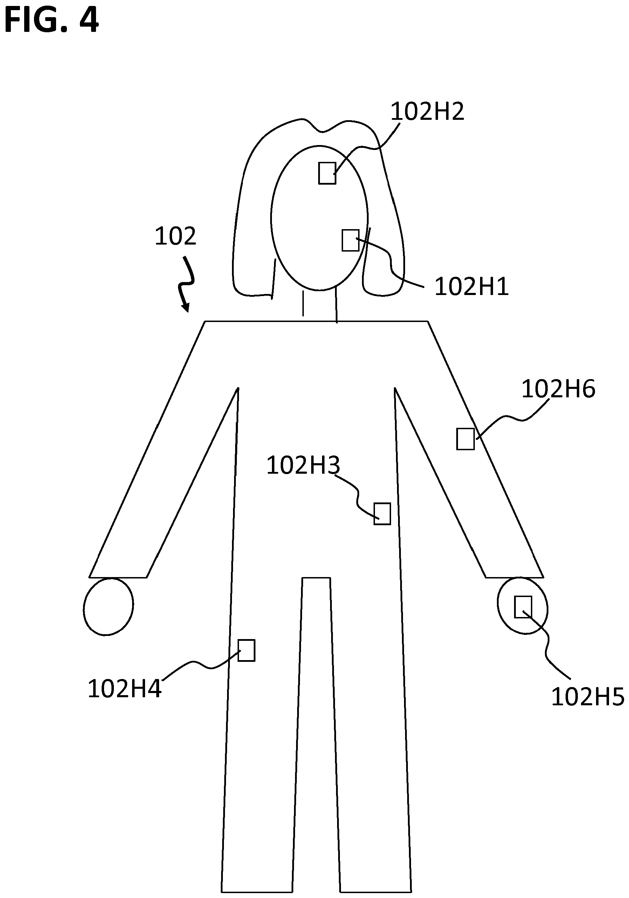

Der Hautfeuchtigkeitsgehalt kann in verschiedenen Ausführungsbeispielen für mindestens einen Hautbereich 102H des Nutzers 102 ermittelt werden. Wie in FIG. 1 und als Hautbereich 102H dargestellt ist, kann in verschiedenen Ausführungsbeispielen der Hautbereich 102H ein Teil einer Gesichtshaut des Nutzers 102 sein. Alternativ oder zusätzlich kann, wie in FIG. 4 dargestellt ist, welche Hautregionen 102H zum Anwenden eines Verfahrens und einer Vorrichtung zum Ermitteln eines Hautfeuchtigkeitsgehalt gemäß verschiedenen Ausführungsbeispielen zeigt, ein anderer bzw. weiterer Hautbereich 102H, beispielsweise an einem Arm (Hautbereich 102H6), einem Bein (Hautbereich 102H4), einem Rumpf (Bereich 102H4) und/oder einer Hand (Bereich 102H5) eines Nutzers 102 zum Ermitteln eines Hautfeuchtigkeitsgehalts dieses Hautbereichs verwendet werden. In verschiedenen Ausführungsbeispielen kann die Hautfeuchtigkeitsmessvorrichtung 106 Teil einer tragbaren Vorrichtung sein, wie beispielhaft in FIG. 1, FIG. 2A, FIG. 2B, FIG. 3A, FIG. 3B und FIG. 3C dargestellt.

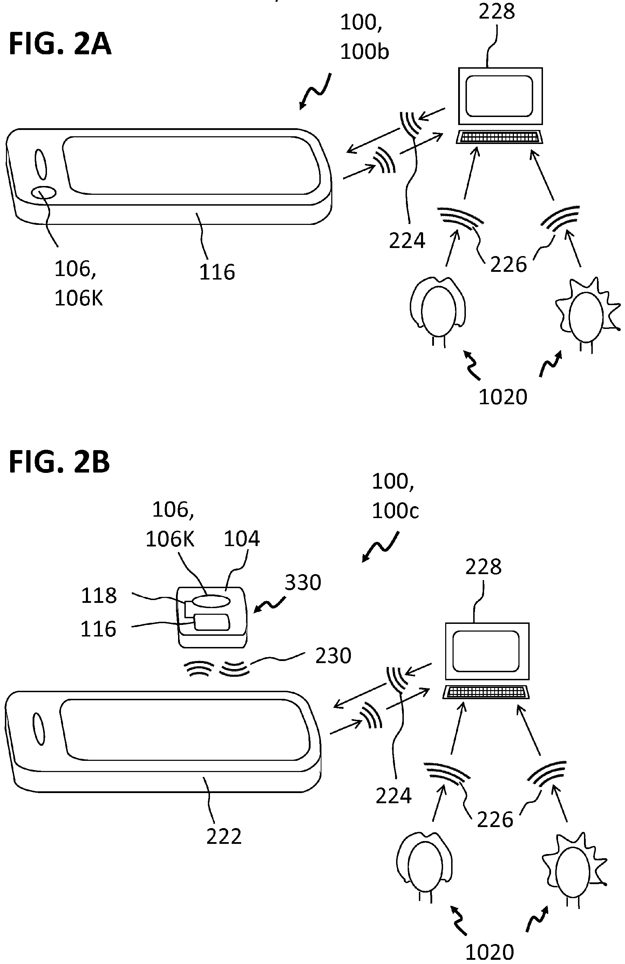

In verschiedenen Ausführungsbeispielen kann die tragbare Vorrichtung ein Smartphone, ein Tablet oder ein iPad sein, wie in FIG. 2A dargestellt, oder beispielsweise ein Messelement 330, wie beispielhaft in FIG. 2B, FIG. 3A, FIG. 3B und FIG. 3C dargestellt, wobei eine weitere externe Datenverarbeitungsvorrichtung 222 Teil der Vorrichtung 100 sein kann, wobei das Messelement 330 mit der weiteren externen Datenverarbeitungsvorrichtung 222 Daten austauschen kann, z.B. mittels einer (z.B. kabellosen) Datenaustauschverbindung 230. (wobei alternativ eine andere Form der Datenübertragung genutzt werden kann, z.B. eine kabelgebundene). Dabei kann eine Kombination aus der integrierten Einheit 330 und der weiteren externen Datenverarbeitungsvorrichtung 222 in verschiedenen Ausführungsbeispielen die Vorrichtung 100c, 100d, 100e, bzw. 100f zum Ermitteln des Hautfeuchtigkeitsgehalts bilden.

Dabei kann in verschiedenen Ausführungsbeispielen die elektronische Schaltkreisvorrichtung 116, welche Teil des Messelements 330 sein kann, im Wesentlichen dafür vorgesehen sein, das Messsignal, welches von der Hautfeuchtigkeitsmessvorrichtung 106 bereitgestellt wird, zu erfassen und der weiteren externen Datenverarbeitungsvorrichtung 222 bereitzustellen, z.B. zum Zuordnen eines Hautfeuchtigkeitsgehalts zum Messsignal. In verschiedenen Ausführungsbeispielen kann die tragbare Vorrichtung an einem Smartphone, einem Tablet oder einem iPad anbringbar sein (nicht dargestellt).

In verschiedenen Ausführungsbeispielen kann das Erfassen des Signals und/oder das Zuordnen des Hautfeuchtigkeitsgehalts zum erfassten Signal mittels einer App erfolgen.

Die elektronische Schaltkreisvorrichtung 116 kann in verschiedenen Ausführungsbeispielen eingerichtet sein, wie oben beschrieben mittels Vergleichens des mittels der Hautfeuchtigkeitsmessvorrichtung 106 erzeugten Signals einen Hautfeuchtigkeitsgehalt der Haut des Nutzers 102 (bei Messung einer Mehrzahl von Hautbereichen 102H den Hautfeuchtigkeitsgehalt des jeweiligen Hautbereiches) zu ermitteln, z.B. einen sehr hohen, mäßig hohen, normalen, mäßig niedrigen oder sehr niedrigen Hautfeuchtigkeitsgehalt. In verschiedenen Ausführungsformen kann der Hautfeuchtigkeitsgehalt auf andere Weise beschrieben oder quantifiziert werden, z.B. als Gewichtsanteil in Prozent oder ähnliches.

Die Vorrichtung 100 zum Ermitteln eines Hautfeuchtigkeitsgehalts gemäß verschiedenen Ausführungsbeispielen kann eine tragbare Vorrichtung 100 sein.

In verschiedenen Ausführungsbeispielen (wie z.B. in FIG. 2A, FIG. 2B, FIG. 3A, FIG. 3B und FIG. 3C für die Vorrichtungen 100b, 100c, 100d, 100e bzw. 100f dargestellt) können die Hautfeuchtigkeitsmessvorrichtung 106 und die elektronische Schaltkreisvorrichtung 116 eine tragbare integrierte Einheit bilden.

In verschiedenen Ausführungsbeispielen kann die integrierte Vorrichtung 100b, 100c, 100d, 100e bzw. 100f eine Größe aufweisen, die es ermöglicht, sie in einer Hand- oder Hosentasche unterzubringen. Beispielsweise kann die integrierte Vorrichtung eine Fläche von weniger als 36 cm² und eine Dicke von weniger als 2 cm aufweisen.

Die elektronische Schaltkreisvorrichtung 116 kann in verschiedenen Ausführungsbeispielen eingerichtet sein, basierend auf dem mittels der Hautfeuchtigkeitsmessvorrichtung 106 gemessenen Signal einen Hautfeuchtigkeitsgehalt zu ermitteln. In verschiedenen Ausführungsbeispielen kann die elektronische Schaltkreisvorrichtung 116 eingerichtet sein, selbst, d.h. direkt, den Hautfeuchtigkeitsgehalt der Haut zu ermitteln.

Für das Ermitteln des Hautfeuchtigkeitsgehalts kann in verschiedenen Ausführungsbeispielen eine Software, beispielsweise eine App, genutzt werden, welche beispielsweise auf einer tragbaren Datenverarbeitungsvorrichtung installiert sein kann, wobei die tragbare Datenverarbeitungsvorrichtung beispielsweise die elektronische Schaltkreisvorrichtung 116 sein kann. In verschiedenen Ausführungsbeispielen kann z.B. die elektronische Schaltkreisvorrichtung 116 aus FIG. 1 bzw. aus FIG. 2A auf diese Weise genutzt werden.

In verschiedenen Ausführungsbeispielen kann die tragbare Datenverarbeitungsvorrichtung die weitere, z.B. externe, Datenverarbeitungsvorrichtung 222 sein, wie z.B. in FIG. 2B, FIG. 3A, FIG. 3B und FIG. 3C dargestellt.

In verschiedenen Ausführungsbeispielen kann der Hautfeuchtigkeitsgehalt zeitlich abhängig (z.B. mehrmals täglich, täglich, wöchentlich, oder mit jeder anderen zeitlichen Abhängigkeit) und/oder unter verschiedenen Umgebungsbedingungen (z.B. in einer Umgebung mit hoher oder niedriger Luftfeuchtigkeit, bei Kälte oder Hitze, usw.) ermittelt werden.

In verschiedenen Ausführungsbeispielen können Kalibrationsmessungen (auch als Referenzmessungen bezeichnet), welche beispielsweise in einem Labor durchgeführt werden können, und deren Ergebnisse in der Datenverarbeitungsvorrichtung gespeichert sein können, beispielsweise als Teil der Software/App, genutzt werden, um ein mittels der Hautfeuchtigkeitsmessvorrichtung messbares Signal, z.B. wie oben beschrieben (z.B. eine Spannung, einen Strom oder einen Widerstand) einem Hautfeuchtigkeitsgehalt zuzuordnen.

In verschiedenen Ausführungsbeispielen kann das Ermitteln des Hautfeuchtigkeitsgehalts, beispielsweise wie oben beschrieben, direkt mittels der tragbaren elektronischen Schaltkreisvorrichtung 116 ausgeführt werden, z.B. mittels der Software/App, welche auf der tragbaren elektronischen Schaltkreisvorrichtung 116 installiert sein kann.

In verschiedenen Ausführungsbeispielen kann das Ermitteln des Hautfeuchtigkeitsgehalts, beispielsweise wie oben beschrieben, indirekt mittels der tragbaren elektronischen Schaltkreisvorrichtung 116 erfolgen, beispielsweise indem die elektronische Schaltkreisvorrichtung 116 das Ermitteln des Hautfeuchtigkeitsgehalts mittels einer weiteren externen Datenverarbeitungsvorrichtung 222 veranlasst. Die elektronische Schaltkreisvorrichtung 116 kann in verschiedenen Ausführungsbeispielen eine relativ einfache elektronische Schaltkreisvorrichtung aufweisen, welche beispielsweise eingerichtet sein kann, im Wesentlichen lediglich das Signal der Hautfeuchtigkeitsmessvorrichtung 106 zu erfassen (es ggf. mit den Referenzsignalen zu vergleichen) und der weiteren externen Datenverarbeitungsvorrichtung 222 zu übertragen. In verschiedenen Ausführungsbeispielen kann die elektronische Schaltkreisvorrichtung 116 eine leistungsfähigere, vielseitige Schaltkreisvorrichtung (z.B. ein Smartphone, ein Tablet oder ein iPad) aufweisen, welches nicht nur eingerichtet sein kann, das Signal der Hautfeuchtigkeitsmessvorrichtung 106 zu erfassen (und ggf. mit den Referenzsignalen zu vergleichen) und der weiteren externen Datenverarbeitungsvorrichtung 222 zu übertragen, sondern zusätzlich eingerichtet sein kann, verschiedene weitere Funktionen auszuführen, z.B. Eingaben vom Nutzer 102 zu erfragen und zu speichern, Ergebnisse darzustellen, ein Foto des Nutzers 102 zum Anzeigen der Ergebnisse bereitzustellen (z.B. mittels einer Kamera) und zu verarbeiten, usw.

In verschiedenen Ausführungsbeispielen können das erfasste Signal und/oder das Vergleichsergebnis von der tragbaren elektronischen Schaltkreisvorrichtung 116, wie in FIG. 1 und FIG. 2A dargestellt, der externen Datenverarbeitungsvorrichtung 228, beispielsweise einem zentralen Computer oder einer Prozessor-Cloud-Architektur (verkürzend auch als "Cloud" bezeichnet), bereitgestellt werden, z.B. an diese übertragen werden. Die externe Datenverarbeitungsvorrichtung 228 kann beispielsweise eine höhere Rechenleistung und/oder eine größere Speicherkapazität als die elektronische Schaltkreisvorrichtung 116 aufweisen. Außerdem kann die externe Datenverarbeitungsvorrichtung 228, beispielsweise wenn sie als Cloud gestaltet ist, ermöglichen, dass die weiteren Nutzer 1020 auf die externe Datenverarbeitungsvorrichtung zugreifen können, z.B. um die Datenbank zu aktualisieren. Die externe Datenverarbeitungsvorrichtung 228 kann eingerichtet sein, den Hautfeuchtigkeitsgehalt aus den bereitgestellten Daten, z.B. dem Signal oder dem Ergebnis des Vergleichs, zu ermitteln, beispielsweise mittels einer Software, z.B. einer App, beispielsweise wie oben beschrieben mittels Vergleichs mit der Datenbank. In verschiedenen Ausführungsbeispielen kann die elektronische Schaltkreisvorrichtung 116 ferner eingerichtet sein, den Hautfeuchtigkeitsgehalt von der weiteren externen Datenverarbeitungsvorrichtung 222 zu empfangen und bereitzustellen, beispielsweise wie oben beschrieben. Zum Datenaustausch mit der weiteren externen Datenverarbeitungsvorrichtung 222 kann die elektronische Schaltkreisvorrichtung 116 in verschiedenen Ausführungsbeispielen eine Vorrichtung zur kabellosen Datenübertragungsvorrichtung aufweisen, z.B. mittels WLAN, Thread, ZigBee oder Bluetooth. In verschiedenen Ausführungsbeispielen kann die elektronische Schaltkreisvorrichtung 116 eingerichtet sein, die Daten mit der weiteren externen Datenverarbeitungsvorrichtung 222 mittels einer Kabelverbindung auszutauschen.

Die Datenbank kann in verschiedenen Ausführungsbeispielen in der tragbaren elektronischen Schaltkreisvorrichtung 116, in der externen Datenverarbeitungsvorrichtung 228 (z.B. der Cloud) und/oder in der weiteren externen Datenverarbeitungsvorrichtung 222 gespeichert sein.

Beim Ermitteln des Hautfeuchtigkeitsgehalts kann derjenige Hautfeuchtigkeitsgehalt als der Hautfeuchtigkeitsgehalt des Nutzers ermittelt werden, für den der Messwert der Hautfeuchtigkeitsmessvorrichtung 106 die geringsten Abweichungen vom in der Datenbank zugeordneten Messwert zeigt.

In verschiedenen Ausführungsbeispielen kann mittels der elektronischen Schaltkreisvorrichtung, z.B. mittels der Software/App, der Hautfeuchtigkeitsgehalt bereitgestellt werden. Der Hautfeuchtigkeitsgehalt kann beispielsweise als Wert (z.B. mit willkürlichen Einheiten), als verbale Mitteilung, als graphische Darstellung, o.ä. mitgeteilt, z.B. dargestellt werden, z.B. mittels Anzeigens, z.B. an einem Bildschirm der tragbaren elektronischen Schaltkreisvorrichtung 116.

Insbesondere in einem Fall, in welchem der Hautfeuchtigkeitsgehalt für eine Mehrzahl von Hautbereichen ermittelt wird, kann das Bereitstellen des Hautfeuchtigkeitsgehalts an den Nutzer eine graphische Darstellung aufweisen, beispielsweise eine Anzeige, z.B. mittels einer Anzeigevorrichtung (z.B. eines Bildschirms/Displays). Dafür kann in verschiedenen Ausführungsbeispielen eine Darstellung eines Körpers oder eines Körperbereichs (z.B. des Gesichts) verwendet werden, beispielsweise als symbolische Darstellung oder als ein Foto des Nutzers 102. Der Darstellung kann eine Veranschaulichung des Hautfeuchtigkeitsgehalts überlagert sein, z.B. mittels unterschiedlicher Symbole/Markierungen/Schraffuren für Bereiche unterschiedlichen Hautfeuchtigkeitsgehalts.

In verschiedenen Ausführungsbeispielen kann die Vorrichtung 100 ferner eingerichtet sein, basierend auf dem ermittelten Hautfeuchtigkeitsgehalt eine kosmetische Hautbehandlungsempfehlung zu ermitteln und dem Nutzer 102 bereitzustellen.

In verschiedenen Ausführungsbeispielen kann/können jedem der Mehrzahl von Hautzuständen mindestens ein geeignetes Pflegeprodukt und/oder mindestens ein Pflegehinweis zugeordnet sein. Die Zuordnung kann beispielsweise experimentell, z.B. bei Laborversuchen, ermittelt worden sein. In verschiedenen Ausführungsbeispielen können zum Beurteilen einer Eignung eines Pflegeprodukts und/oder eines Pflegehinweises zum Pflegen einer Haut mit einem gegebenen Hautfeuchtigkeitsgehalt Literaturdaten zu Grunde gelegt werden.

In verschiedenen Ausführungsbeispielen kann jedem der Hautfeuchtigkeitsgehalte ein Qualitätswert zugeordnet sein oder werden. Ein Pflegeprodukt und/oder ein Pflegehinweis kann für einen Hautfeuchtigkeitsgehalt als geeignet bewertet werden, wenn zu erwarten ist, z.B. aufgrund von Literaturdaten, Versuchsergebnissen oder Erfahrungswerten, dass bei einer (z.B. regelmäßigen) Anwendung des Pflegeprodukts und/oder des Pflegehinweises der Hautfeuchtigkeitsgehalt des Nutzers 102 aufrechterhalten wird oder sich zu einem Hautfeuchtigkeitsgehalt mit einem höheren Qualitätswert (z.B. hin zu einem normalen Hautfeuchtigkeitsgehalt) ändert.

In verschiedenen Ausführungsbeispielen kann eine Beurteilung einer Eignung eines Pflegeproduktes, einen Hautfeuchtigkeitsgehalt zu verbessern, bestätigt oder abgeändert werden mittels eines Aufnehmens von Erfahrungswerten weiterer Nutzer 1020 mit demselben oder einem ähnlichen Hautfeuchtigkeitsgehalt, beispielsweise von Erfahrungswerten hinsichtlich eines Behandlungserfolgs, ähnlich dem, was in FIG. 1, FIG. 2A, FIG. 2B und FIG. 3B für das Aktualisieren der in der Prozessor-Cloud-Architektur 228 gespeicherten Datenbank zum Ermitteln des Hautfeuchtigkeitsgehalts durch die weiteren Nutzer 1020 dargestellt ist. Die Erfahrungswerte können von den weiteren Nutzern 1020 beispielsweise der externen Datenverarbeitungsvorrichtung 228 bereitgestellt werden, beispielsweise mittels einer kabellosen Datenübertragung 226. Alternativ kann auch eine Übertragung der Daten mittels Kabel genutzt werden. Anhand der Erfahrungswerte kann die Datenbank in der weiteren externen Datenverarbeitungsvorrichtung 222 und/oder in der tragbaren elektronischen Schaltkreisvorrichtung 116 aktualisiert werden. Damit kann ermöglicht sein, dass der Nutzer 102 immer eine optimale Empfehlung erhält.

In verschiedenen Ausführungsbeispielen können beim Ermitteln der Produkt- und/oder Pflegeempfehlungen ferner Informationen hinsichtlich eines generellen Gesundheitszustands, eines Hautzustands, Ernährungsgewohnheiten und weiterer Verhaltensweisen des Nutzers (z.B. tägliche Aufenthaltsdauer im Freien/in der Sonne/im Wasser, Rauchgewohnheiten, Sportgewohnheiten, usw.) verwendet werden, z.B. mittels der auf der tragbaren elektronischen Schaltkreisvorrichtung 116 und/oder auf der weiteren externen Datenverarbeitungsvorrichtung 222 installierten Software/App. Die Informationen können in verschiedenen Ausführungsbeispielen mittels der tragbaren elektronischen Schaltkreisvorrichtung 116 vom Nutzer 102 erfragt werden, welcher sie in die elektronische Schaltkreisvorrichtung 116 eingeben kann, beispielsweise mittels einer Tastatur, als Sprachnachricht, als Auswahl aus einem von der tragbaren elektronischen Schaltkreisvorrichtung 116 dargestellten Menü, oder ähnliches.

Beispielsweise können, wenn der Nutzer 102 als zusätzliche Information zur Verfügung stellt, dass er viel Zeit im Wasser oder im Freien verbringt, bei der Produkt- und/oder Pflegehinweisempfehlung basierend auf dem Hautfeuchtigkeitsgehalt des Nutzers 102 beispielsweise diejenigen zugeordneten Pflegeprodukte dem Nutzer 102 empfohlen werden, die nicht nur für seinen Hautfeuchtigkeitsgehalt geeignet sind, sondern außerdem z.B. wasserfest und/oder mit einem UV-Filter versehen sind.

In verschiedenen Ausführungsbeispielen kann die Produktempfehlung und/oder der Pflegehinweis dem Nutzer 102 bereitgestellt werden, beispielsweise mittels der elektronischen Schaltkreisvorrichtung 116, z.B. mittels des Bildschirms der elektronischen Schaltkreisvorrichtung 116. Beispielsweise kann eine graphische Darstellung durch (z.B. Text-) Mitteilungen ergänzt werden, z.B. die ermittelten Produktempfehlungen und/oder Pflegehinweise.

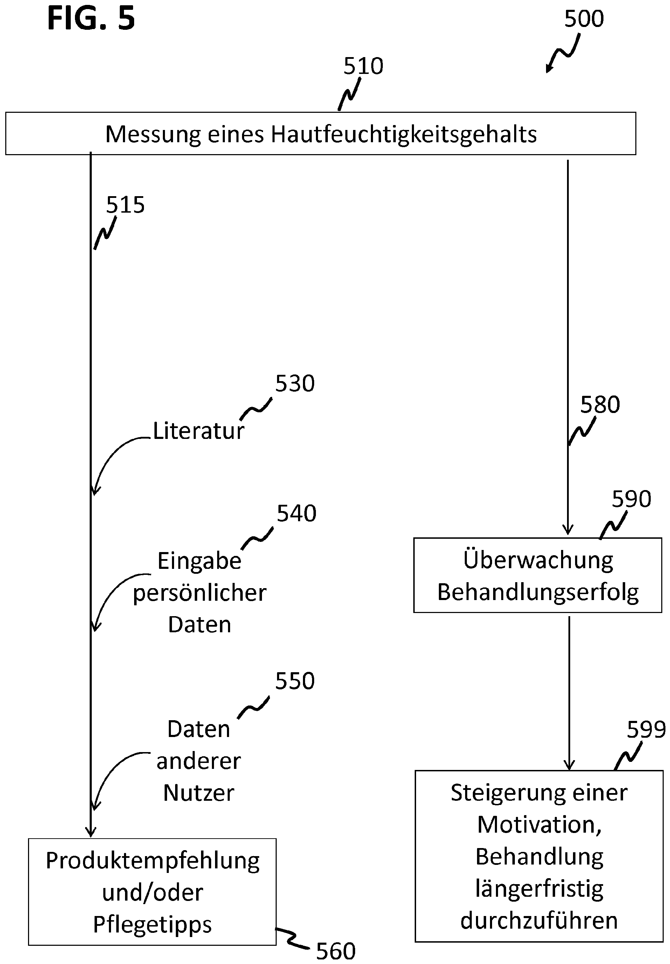

FIG. 5 zeigt ein Diagramm 500 zum Veranschaulichen eines Verfahrens zum Ermitteln eines Kosmetikprodukts zur Hautbehandlung gemäß verschiedenen Ausführungsbeispielen. FIG. 5 kann im Wesentlichen diejenigen Prozesse veranschaulichen, die an anderer Stelle im Zusammenhang mit dem Verfahren zum Ermitteln eines Kosmetikprodukts zur Hautbehandlung gemäß verschiedenen Ausführungsbeispielen beschrieben sind.

Das Verfahren kann gemäß verschiedenen Ausführungsbeispielen aufweisen ein Ermitteln eines Hautfeuchtigkeitsgehalts (bei 510), beispielsweise wie oben beschrieben.

Basierend auf dem ermittelten Hautfeuchtigkeitsgehalt kann eine Produkt- oder Pflegetippempfehlung ermittelt werden (bei 560). In verschiedenen Ausführungsbeispielen (als Pfad 515 gekennzeichnet) können zusätzlich zum Hautfeuchtigkeitsgehalt auch Literaturdaten (mit 530 gekennzeichnet), die persönlichen Daten (z.B. wie oben beschrieben, mit 540 gekennzeichnet), und/oder Daten anderer Nutzer, z.B. deren Erfahrungswerte (als 550 gekennzeichnet) hinzugezogen werden.

In verschiedenen Ausführungsbeispielen kann ferner, wie bei Pfad 580 dargestellt, mittels eines Ermittelns des Hautfeuchtigkeitsgehalts (bei 510) ein Behandlungserfolg überwacht werden (bei 590). Dies kann beispielsweise besonders während und nach einer kosmetischen Behandlung aufgrund der Produktempfehlung (bei 560) sinnvoll sein. Ein mittels des Verfahrens zum Ermitteln des Hautfeuchtigkeitsgehalts anhand objektiver Werte belegter Behandlungserfolg kann eine Motivation beim Nutzer steigern (bei 599).

In verschiedenen Ausführungsbeispielen können, wie oben beschrieben, anhand des ermittelten Hautfeuchtigkeitsgehalts Produktempfehlungen für individuell für den Nutzer 102 passende kosmetische Produkte und/oder individuelle Pflegehinweise abgeleitet werden. Die Produktempfehlungen und/oder Pflegehinweise können beispielsweise mittels einer Software, z.B. einer App, bereitgestellt werden.

Dem Nutzer 102 kann ein empfohlenes kosmetisches Produkt direkt zum Kauf angeboten werden und der Nutzer 102 kann über eine Eingabe den Kauf in die Wege leiten. Neben dem Kauf von empfohlenen kosmetischen Produkten können dem Nutzer auch weitergehende Informationen zum käuflichen Erwerb angeboten werden. Diese weitergehenden Informationen können sich auf detailliertere Pflegehinweise beziehen. Eine Software/App empfängt beispielsweise die Anfrage, dass der Nutzer 102 das kosmetische Produkt erwerben möchte, speichert die Anfrage und/oder übermittelt die Anfrage an ein Handelsunternehmen, welches die kosmetischen Produkte vertreibt. Der Nutzer 102 kann durch die Software/App aufgefordert werden, seine persönlichen Daten (Adresse, Bankinformationen, Versendungspräferenz, etc.) über die Eingabeeinheit einzugeben. Alternativ kann dem Nutzer angegeben werden, wo (zum Beispiel Drogeriemarkt, Apotheke, etc.) er das Mundhygieneprodukt erwerben kann.

In verschiedenen Ausführungsbeispielen kann dem Nutzer 102 zum Einsatz von Kosmetikprodukten, die für den Nutzer individuell hergestellt werden, zugeraten und ein Bestellvorgang, vorzugsweise durch Aufrufen einer Internetseite eines Herstellers von individuellen Kosmetikprodukten, eingeleitet werden.

Immer mehr Nutzer wünschen sich ein individuell auf ihre Bedürfnisse abgestimmtes Produkt. Dabei kann es sich um ein speziell für den Nutzer hergestelltes Produkt oder ein sogenanntes "mass customized" Produkt handeln. Bei einem "mass customized" Produkt kann durch Variation von wenigen, aus Nutzersicht jedoch entscheidenden Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/lnhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurator abläuft. Dieser Konfigurator hilft dem Nutzer bei der Auswahl der Merkmale/Inhaltsstoffe und weist ihn auf die zulässigen/unzulässigen Merkmalskombinationen hin, wobei letztere dann nicht ausgewählt werden können.

Bei Produkten für die Haut umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten Hautfeuchtigkeitsgehalt ungeeigneter Inhaltsstoffe vermieden werden. Umgekehrt kann die Auswahl für den ermittelten Hautfeuchtigkeitsgehalt geeigneter Inhaltsstoffe durch den Produktkonfigurator vorgegeben oder vorgeschlagen werden.

In verschiedenen Ausführungsbeispielen kann die Software/App, welche den Hautfeuchtigkeitsgehalt der Haut ermittelt, dieselbe sein, die die Produktempfehlung und/oder die Pflegehinweise ermittelt und/oder den Bestellvorgang einleitet. In verschiedenen Ausführungsbeispielen können unterschiedliche Softwareprogramme/Apps verwendet werden für einen Teil der verschiedenen Vorgänge oder alle verschiedenen Vorgänge (Ermitteln des Hautfeuchtigkeitsgehalts, Ermitteln einer Produktempfehlung, Ermitteln eines Pflegehinweises, Einleitung eines Bestellvorgangs).

In verschiedenen Ausführungsbeispielen kann der Besuch eines Hautarztes oder eines Kosmetikers empfohlen werden. In verschiedenen Ausführungsbeispielen kann direkt über die Software/App, welche den Hautfeuchtigkeitsgehalt und/oder die Produktempfehlung und/oder die Pflegehinweise ermittelt und/oder einen Bestellvorgang einleitet, ein Buchungsvorgang eingeleitet werden. Dazu können beispielsweise in der Software/App die Kontaktdaten von Hautärzten und/oder Kosmetikern hinterlegt sein und diese dem Nutzer angezeigt werden. Zusätzlich kann über Filter, wie beispielsweise die Postleitzahl, die Auswahl eingeschränkt werden. In verschiedenen Ausführungsbeispielen kann direkt über die Software/App eine Terminbuchung vorgenommen werden. Alternativ kann die Buchung eines Hautarzttermins und/oder eines Kosmetiktermins über eine separate Software/App, wie beispielsweise Treatwell, erfolgen.

In verschiedenen Ausführungsbeispielen kann ein Behandlungserfolg bei einer kosmetischen Behandlung, welche ein positives Beeinflussen des ermittelten Hautfeuchtigkeitsgehalts zum Ziel haben kann, überwacht werden. In verschiedenen Ausführungsbeispielen kann die Software/App eine Kontrolle und/oder Nachverfolgung der Ergebnisse mittels einer Darstellung (z.B. einer graphischen Darstellung) der Messergebnisse im Verlauf der Zeit ermöglichen.

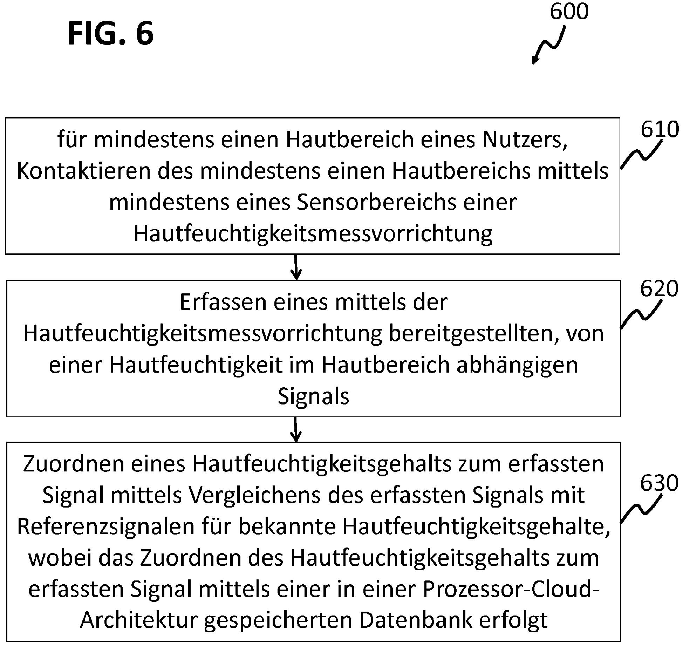

FIG. 6 zeigt ein Flussdiagramm 600 eines Verfahrens zum Ermitteln eines Hautfeuchtigkeitsgehalts gemäß verschiedenen Ausführungsbeispielen.

Es wird ein Verfahren zum Ermitteln eines Hautfeuchtigkeitsgehalts bereitgestellt. Das Verfahren weist auf:
ein Kontaktieren des mindestens einen Hautbereichs mittels mindestens eines Sensorbereichs einer Hautfeuchtigkeitsmessvorrichtung für mindestens einen Hautbereich eines Nutzers (bei 610), Erfassen eines mittels der Hautfeuchtigkeitsmessvorrichtung bereitgestellten, von einer Hautfeuchtigkeit im Hautbereich abhängigen Signals (bei 620) und Zuordnen eines Hautfeuchtigkeitsgehalts zum erfassten Signal mittels Vergleichens des erfassten Signals mit Referenzsignalen für bekannte Hautfeuchtigkeitsgehalte, wobei das Zuordnen des Hautfeuchtigkeitsgehalts zum erfassten Signal mittels einer in einer Prozessor-Cloud-Architektur gespeicherten Datenbank erfolgt (bei 630).

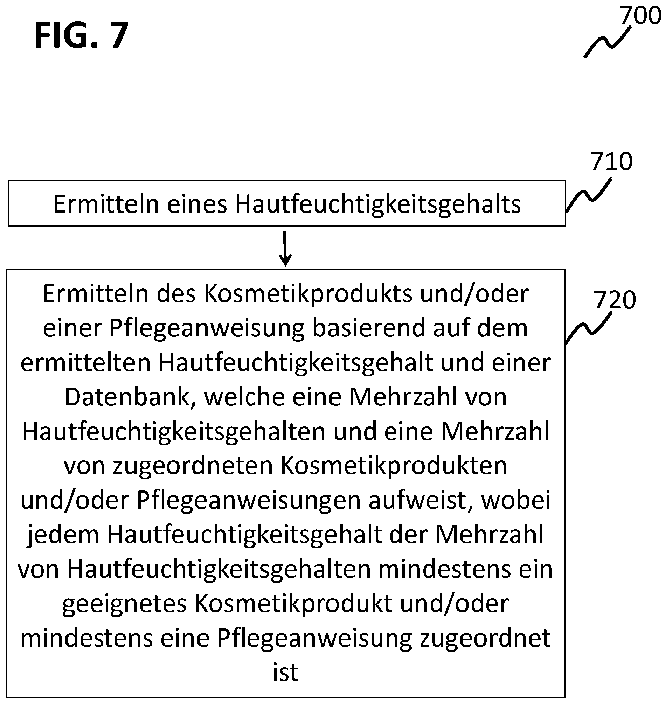

FIG. 7 zeigt ein Flussdiagramm 700 eines Verfahrens zum Ermitteln einer kosmetischen Hautbehandlungsempfehlung gemäß verschiedenen Ausführungsbeispielen.

In verschiedenen Ausführungsbeispielen wird ein Verfahren zum Ermitteln einer kosmetischen Hautbehandlungsempfehlung bereitgestellt. Das Verfahren kann aufweisen: ein Ermitteln eines Hautfeuchtigkeitsgehalts gemäß verschiedenen Ausführungsbeispielen (bei 710) und ein Ermitteln des Kosmetikprodukts und/oder der Pflegeanweisung basierend auf dem ermittelten Hautfeuchtigkeitsgehalt und einer Datenbank, welche eine Mehrzahl von Hautfeuchtigkeitsgehalten und eine Mehrzahl von zugeordneten Kosmetikprodukten bzw. Pflegeanweisungen aufweist, wobei jedem Hautfeuchtigkeitsgehalt der Mehrzahl von Hautfeuchtigkeitsgehalten mindestens ein geeignetes Kosmetikprodukt und/oder mindestens eine geeignete Pflegeanweisung zugeordnet sein kann (bei 720).

Manche der Ausführungsbeispiele sind im Zusammenhang mit Vorrichtungen beschrieben, und manche der Ausführungsbeispiele sind im Zusammenhang mit Verfahren beschrieben. Weitere vorteilhafte Ausgestaltungen des Verfahrens ergeben sich aus der Beschreibung der Vorrichtung und umgekehrt.

## Patentansprüche

1. Verfahren zum Ermitteln eines Hautfeuchtigkeitsgehalts, aufweisend:
für mindestens einen Hautbereich (102H) eines Nutzers (102), Kontaktieren des mindestens einen Hautbereichs mittels mindestens eines Sensorkontaktbereichs (102K) einer Hautfeuchtigkeitsmessvorrichtung (106); Erfassen eines mittels der Hautfeuchtigkeitsmessvorrichtung bereitgestellten, von einer Hautfeuchtigkeit im Hautbereich abhängigen Signals;
Zuordnen eines Hautfeuchtigkeitsgehalts zum erfassten Signal mittels Vergleichens des erfassten Signals mit Referenzsignalen für bekannte Hautfeuchtigkeitsgehalte, wobei das Zuordnen des Hautfeuchtigkeitsgehalts zum erfassten Signal mittels einer in einer Prozessor-Cloud-Architektur (228) gespeicherten Datenbank erfolgt;
wobei der mindestens eine Sensorkontaktbereich eine Mehrzahl von voneinander beabstandeten Sensorkontaktbereichen aufweist und wobei das Verfahren ferner aufweist:
Bilden eines Signal-Mittelwerts aus der Mehrzahl von Signalen der Mehrzahl von Sensorkontaktbereichen;
wobei in einem Abstandsbereich zwischen den Sensorkontaktbereichen ein Feuchtigkeit aufnehmende Material angeordnet ist.

2. Verfahren gemäß Anspruch 1, ferner aufweisend:
Aktualisieren der Datenbank basierend auf neuen Zuordnungen des Hautfeuchtigkeitsgehalts zum erfassten Signal von einer Mehrzahl von weiteren Nutzern.

3. Verfahren gemäß Anspruch 1 oder 2,
wobei die Hautfeuchtigkeitsmessvorrichtung mindestens eine aufweist aus einer Gruppe von Hautfeuchtigkeitsmessvorrichtungen, wobei die Gruppe aufweist:
eine kapazitive Hautfeuchtigkeitsmessvorrichtung;
eine Impedanz-Hautfeuchtigkeitsmessvorrichtung; eine Hautfeuchtigkeitsmessvorrichtung, welche den elektrischen Leitwert der Haut nutzt, und
eine Hautfeuchtigkeitsmessvorrichtung, welche eine transiente Wärmeübertragung der Haut nutzt.

4. Verfahren gemäß einem der Ansprüche 1 bis 3,
wobei nach dem Kontaktieren des mindestens einen Hautbereichs mittels des mindestens einen Sensorkontaktbereichs der Hautfeuchtigkeitsmessvorrichtung das Erfassen des Signals und das Zuordnen des Hautfeuchtigkeitsgehalts zum erfassten Signal mehrmals mit zeitlichem Abstand oder kontinuierlich ausgeführt wird.

5. Verfahren gemäß einem der Ansprüche 1 bis 4,
wobei der mindestens eine Hautbereich eine Mehrzahl von Hautbereichen des Nutzers aufweist.

6. Verfahren gemäß einem der Ansprüche 1 bis 5,
wobei die Hautfeuchtigkeitsmessvorrichtung Teil einer tragbaren Vorrichtung ist.

7. Verfahren zum Ermitteln einer kosmetischen Hautbehandlungsempfehlung, aufweisend:
Ermitteln eines Hautfeuchtigkeitsgehalts gemäß einem der Ansprüche 1 bis 6; und
Ermitteln des Kosmetikprodukts und/oder einer Pflegeanweisung basierend auf dem ermittelten Hautfeuchtigkeitsgehalt und einer in der Prozessor-Cloud-Architektur gespeicherten weiteren Datenbank, welche eine Mehrzahl von Hautfeuchtigkeitsgehalten und eine Mehrzahl von zugeordneten Kosmetikprodukten und/oder Pflegeanweisungen aufweist, wobei jedem Hautfeuchtigkeitsgehalts der Mehrzahl von Hautfeuchtigkeitsgehalten mindestens ein geeignetes Kosmetikprodukt und/oder mindestens eine Pflegeanweisung zugeordnet ist.

8. Verfahren gemäß Anspruch 7,
wobei das Kosmetikprodukt und/oder die Pflegeanweisung geeignet ist für den Hautfeuchtigkeitsgehalt, wenn basierend auf in der weiteren Datenbank gespeicherten Erfahrungswerten einer Mehrzahl weiterer Nutzer mit dem Kosmetikprodukt eine Normalisierung des Hautfeuchtigkeitsgehalts zu erwarten ist.

9. Verfahren gemäß einem der Ansprüche 7 oder 8,
wobei das Ermitteln des Hautfeuchtigkeitsgehalts und/oder das Ermitteln der kosmetischen Hautbehandlungsempfehlung ein Übertragen des Signals und/oder des Hautfeuchtigkeitsgehalts an eine externe Datenverarbeitungsvorrichtung und ein Empfangen des Hautfeuchtigkeitsgehalts und/oder der kosmetischen Hautbehandlungsempfehlung aufweist.

10. Vorrichtung (100) zum Ermitteln eines Hautfeuchtigkeitsgehalts, aufweisend:
eine Hautfeuchtigkeitsmessvorrichtung (106), welche mindestens einen Sensorkontaktbereich (102K) aufweist und eingerichtet ist, bei einem Kontaktieren mindestens eines Hautbereichs (102H) eines Nutzers (102) mittels des mindestens einen Sensorkontaktbereichs ein von einer Hautfeuchtigkeit im Hautbereich abhängiges Signal bereitzustellen;
eine elektronische Schaltkreisvorrichtung (116) zum Erfassen des von der Hautfeuchtigkeitsmessvorrichtung bereitgestellten Signals und zum Zuordnen eines Hautfeuchtigkeitsgehalts zum erfassten Signal mittels Vergleichens des erfassten Signals mit Referenzsignalen für bekannte Hautfeuchtigkeitsgehalte, wobei das Zuordnen des Hautfeuchtigkeitsgehalts zum erfassten Signal mittels einer in einer Prozessor-Cloud-Architektur (228) gespeicherten Datenbank erfolgt,
wobei der mindestens eine Sensorkontaktbereich eine Mehrzahl von voneinander beabstandeten Sensorkontaktbereichen(106K) aufweist,
wobei die elektronische Schaltkreisvorrichtung eingerichtet ist, einen Signal-Mittelwerts aus der Mehrzahl von Signalen der Mehrzahl von Sensorkontaktbereichen zu bilden und
wobei in einem Abstandsbereich zwischen den Sensorkontaktbereichen ein Feuchtigkeit aufnehmende Material angeordnet ist.

11. Vorrichtung gemäß Anspruch 10,
wobei die Hautfeuchtigkeitsmessvorrichtung mindestens eine aufweist aus einer Gruppe von Hautfeuchtigkeitsmessvorrichtungen, wobei die Gruppe aufweist:
eine kapazitive Hautfeuchtigkeitsmessvorrichtung;
eine Impedanz-Hautfeuchtigkeitsmessvorrichtung; eine Hautfeuchtigkeitsmessvorrichtung, welche den elektrischen Leitwert der Haut nutzt; und
eine Hautfeuchtigkeitsmessvorrichtung, welche eine transiente Wärmeübertragung der Haut nutzt.

12. Vorrichtung gemäß einem der Ansprüche 10 oder 11, ferner aufweisend:
einen atmungsaktiven Träger (104), wobei die Hautfeuchtigkeitsmessvorrichtung und die elektronische Schaltkreisvorrichtung auf dem atmungsaktiven Träger angeordnet sind.

13. Vorrichtung gemäß einem der Ansprüche 10 bis 12, ferner aufweisend:
eine auf dem atmungsaktiven Träger angeordnete kabellose Datenaustauschvorrichtung zum Austauschen von Daten zwischen der elektronischen Schaltkreisvorrichtung und der Prozessor-Cloud-Architektu r.

## Claims

1. A method for determining a skin moisture content, comprising:
for at least one skin region (102H) of a user (102), contacting the at least one skin region by means of at least one sensor contact region (102K) of a skin moisture measuring device (106);
detecting a signal that is provided by means of the skin moisture measuring device and is dependent on skin moisture in the skin region;
associating a skin moisture content with the detected signal by comparing the detected signal with reference signals for known skin moisture contents, wherein the skin moisture content is associated with the detected signal by means of a database stored in a processor cloud architecture (228);
wherein the at least one sensor contact region comprises a plurality of sensor contact regions that are spaced apart from each other, and wherein the method further comprises:
forming an average signal value from the plurality of signals from the plurality of sensor contact regions;
wherein a moisture-absorbing material is arranged in a spacing region between the sensor contact regions.

2. The method according to claim 1, further comprising:
updating the database on the basis of new associations of the skin moisture content with the detected signal from a plurality of other users.

3. The method according to claim 1 or 2,
wherein the skin moisture measuring device comprises at least one of a group of skin moisture measuring devices, wherein the group comprises:
a capacitive skin moisture measuring device;
an impedance skin moisture measuring device;
a skin moisture measuring device that uses the electrical conductivity of the skin, and
a skin moisture measuring device that uses a transient heat transfer of the skin.

4. The method according to one of claims 1 to 3,
wherein, after contacting the at least one skin region by means of the at least one sensor contact region of the skin moisture measuring device, the detection of the signal and the association of the skin moisture content with the detected signal is carried out several times at intervals or continuously.

5. The method according to one of claims 1 to 4,
wherein the at least one skin region comprises a plurality of skin regions of the user.

6. The method according to one of claims 1 to 5,
wherein the skin moisture measuring device is part of a wearable device.

7. A method for determining a cosmetic skin treatment recommendation, comprising:
determining a skin moisture content according to one of claims 1 to 6; and
determining the cosmetic product and/or a care instruction on the basis of the determined skin moisture content and a further database stored in the processor cloud architecture, which database comprises a plurality of skin moisture levels and a plurality of associated cosmetic products and/or care instructions, wherein each skin moisture content of the plurality of skin moisture contents is associated with at least one suitable cosmetic product and/or at least one care instruction.

8. The method according to claim 7,
wherein the cosmetic product and/or the care instruction is suitable for the skin moisture content if a normalization of the skin moisture content is to be expected with the cosmetic product, based on empirical values of a plurality of other users stored in the further database.

9. The method according to one of claims 7 or 8,
wherein determining the skin moisture content and/or determining the cosmetic skin treatment recommendation comprises transmitting the signal and/or the skin moisture content to an external data processing device and receiving the skin moisture content and/or the cosmetic skin treatment recommendation.

10. A device (100) for determining a skin moisture content, comprising:
a skin moisture measuring device (106) that comprises at least one sensor contact region (102K) and is designed to provide a signal dependent on skin moisture in the skin region when at least one skin region (102H) of a user (102) is contacted by means of the at least one sensor contact region;
an electronic circuit device (116) for detecting the signal provided by the skin moisture measuring device and for associating a skin moisture content with the detected signal by comparing the detected signal with reference signals for known skin moisture levels, wherein the skin moisture content is associated with the detected signal by means of a database stored in a processor cloud architecture (228),
wherein the at least one sensor contact region comprises a plurality of sensor contact regions (106K) that are spaced apart from each other,
wherein the electronic circuit device is designed to form a signal average value from the plurality of signals from the plurality of sensor contact regions, and
wherein a moisture-absorbing material is arranged in a spacing region between the sensor contact regions.

11. The device according to claim 10,
wherein the skin moisture measuring device comprises at least one of a group of skin moisture measuring devices, wherein the group comprises:
a capacitive skin moisture measuring device;
an impedance skin moisture measuring device; a skin moisture measuring device that uses the electrical conductivity of the skin; and
a skin moisture measuring device that uses a transient heat transfer of the skin.

12. The device according to one of claims 10 or 11, further comprising:
a breathable carrier (104), wherein the skin moisture measuring device and the electronic circuit device are arranged on the breathable carrier.

13. The device according to one of claims 10 to 12, further comprising:
a wireless data exchange device arranged on the breathable carrier for exchanging data between the electronic circuit device and the processor cloud architecture.

## Revendications

1. Procédé permettant de déterminer une teneur en humidité de la peau, présentant :
pour au moins une zone de peau (102H) d'un utilisateur (102), la mise en en contact de l'au moins une zone de peau au moyen d'au moins une zone de contact de capteur (102K) d'un dispositif de mesure de l'humidité de la peau (106) ; la détection d'un signal fourni par le dispositif de mesure de l'humidité de la peau et dépendant de l'humidité de la peau dans la zone de peau ;
l'association d'une teneur en humidité de la peau au signal détecté par la comparaison du signal détecté à des signaux de référence pour des teneurs connues en humidité de la peau, l'association de la teneur en humidité de la peau au signal détecté étant effectuée à l'aide d'une base de données stockée dans une architecture en nuage de processeur (228) ;
dans lequel l'au moins une zone de contact de capteur présente une pluralité de zones de contact de capteur écartées les unes des autres et dans lequel le procédé présente en outre : la formation d'une valeur moyenne de signal à partir de la pluralité de signaux de la pluralité de zones de contact de capteur ;
un matériau absorbant l'humidité étant disposé dans une zone d'écartement entre les zones de contact de capteur.

2. Procédé selon la revendication 1, présentant en outre :
la mise à jour de la base de données sur la base de nouvelles associations de la teneur en humidité de la peau au signal détecté d'une pluralité d'autres utilisateurs.

3. Procédé selon la revendication 1 ou 2,
dans lequel le dispositif de mesure de l'humidité de la peau présente au moins un dispositif de mesure de l'humidité de la peau provenant d'un groupe de dispositifs de mesure de l'humidité de la peau, le groupe présentant :
un dispositif de mesure capacitive de l'humidité de la peau ;
un dispositif de mesure par impédance de l'humidité de la peau ;
un dispositif de mesure de l'humidité de la peau qui utilise la conductance électrique de la peau et
un dispositif de mesure de l'humidité de la peau qui utilise un transfert thermique transitoire de la peau.

4. Procédé selon l'une quelconque des revendications 1 à 3,
dans lequel, après la mise en contact de l'au moins une zone de peau au moyen de l'au moins une zone de contact de capteur du dispositif de mesure de l'humidité de la peau, la détection du signal et l'association de la teneur en humidité de la peau au signal détecté sont réalisées plusieurs fois à des intervalles temporels ou en continu.

5. Procédé selon l'une quelconque des revendications 1 à 4,
dans lequel l'au moins une zone de peau présente une pluralité de zones de peau de l'utilisateur.

6. Procédé selon l'une quelconque des revendications 1 à 5,
dans lequel le dispositif de mesure de l'humidité de la peau fait partie d'un dispositif portable.

7. Procédé permettant de déterminer une recommandation de traitement cosmétique de la peau, présentant :
la détermination d'une teneur en humidité de la peau selon l'une quelconque des revendications 1 à 6 ; et
la détermination du produit cosmétique et/ou des instructions de soin sur la base de la teneur en humidité de la peau déterminée et d'une autre base de données stockée dans l'architecture en nuage de processeur, laquelle base de données présente une pluralité de teneurs en humidité de la peau et une pluralité de produits cosmétiques et/ou d'instructions de soin associé(e)s, au moins un produit cosmétique et/ou au moins une instruction de soin approprié(e)(s) étant associé(e)(s) à chaque teneur en humidité de la peau de la pluralité de teneurs en humidité de la peau.

8. Procédé selon la revendication 7,
dans lequel le produit cosmétique et/ou les instructions de soin est/sont adapté(e)(s) à la teneur en humidité de la peau lorsqu'une normalisation de la teneur en humidité de la peau est à prévoir sur la base de valeurs empiriques, stockées dans l'autre base de données, d'une pluralité d'autres utilisateurs avec le produit cosmétique.

9. Procédé selon l'une quelconque des revendications 7 ou 8,
dans lequel la détermination de la teneur en humidité de la peau et/ou la détermination de la recommandation de traitement cosmétique de la peau présente(nt) une transmission du signal et/ou de la teneur en humidité de la peau à un dispositif de traitement de données externe et une réception de la teneur en humidité de la peau et/ou de la recommandation de traitement cosmétique de la peau.

10. Dispositif (100) permettant de déterminer une teneur en humidité de la peau, présentant :
un dispositif de mesure de l'humidité de la peau (106) qui présente au moins une zone de contact de capteur (102K) et qui est conçu pour fournir un signal dépendant de l'humidité de la peau dans la zone de peau lors de la mise en contact d'au moins une zone de peau (102H) d'un utilisateur (102) au moyen de l'au moins une zone de contact de capteur ;
un dispositif de circuit électronique (116) permettant de détecter le signal fourni par le dispositif de mesure de l'humidité de la peau et d'associer une teneur en humidité de la peau au signal détecté par comparaison du signal détecté à des signaux de référence pour des teneurs en humidité de la peau connues, l'association de la teneur en humidité de la peau au signal détecté étant effectuée au moyen d'une base de données stockée dans une architecture en nuage de processeur (228),
dans lequel l'au moins une zone de contact de capteur présente une pluralité de zones de contact de capteur (106K) écartées les unes des autres,
dans lequel le dispositif de circuit électronique est conçu pour former une valeur moyenne de signal à partir de la pluralité de signaux de la pluralité de zones de contact de capteur et
un matériau absorbant l'humidité étant disposé dans une zone d'écartement entre les zones de contact de capteur.

11. Dispositif selon la revendication 10,
dans lequel le dispositif de mesure de l'humidité de la peau présente au moins un dispositif de mesure de l'humidité de la peau provenant d'un groupe de dispositifs de mesure de l'humidité de la peau, le groupe présentant :
un dispositif de mesure capacitive de l'humidité de la peau ;
un dispositif de mesure par impédance de l'humidité de la peau ;
un dispositif de mesure de l'humidité de la peau qui utilise la conductance électrique de la peau ; et
un dispositif de mesure de l'humidité de la peau qui utilise un transfert thermique transitoire de la peau.

12. Dispositif selon l'une quelconque des revendications 10 ou 11, présentant en outre :
un support respirant (104), le dispositif de mesure de l'humidité de la peau et le dispositif de circuit électronique étant disposés sur le support respirant.

13. Dispositif selon l'une quelconque des revendications 10 à 12, présentant en outre :
un dispositif d'échange de données sans fil disposé sur le support respirant et permettant d'échanger des données entre le dispositif de circuit électronique et l'architecture en nuage de processeur.
